(19) Europäisches Patentamt
European Patent Office
Office européen des brevets

(11) EP 0 623 683 B1

(12) **EUROPEAN PATENT SPECIFICATION**

(45) Date of publication and mention
of the grant of the patent:
**27.09.2000 Bulletin 2000/39**

(51) Int. Cl.[7]: **C12Q 1/68**, C12N 15/00

(21) Application number: **94200266.8**

(22) Date of filing: **17.06.1988**

(54) **Transcription-based nucleic acid amplification/detection systems**

Transkriptionbasierte Nukleinsäure-Verstärkungs/Nachweissysteme

Systèmes d'amplification/détection d'acides nucléiques à base de transcription

(84) Designated Contracting States:
**AT BE CH DE FR GB IT LI LU NL SE**

(30) Priority: **19.06.1987 US 64141**
**06.06.1988 US 202978**

(43) Date of publication of application:
**09.11.1994 Bulletin 1994/45**

(62) Document number(s) of the earlier application(s) in
accordance with Art. 76 EPC:
**88906601.5 / 0 368 906**

(73) Proprietor: **Akzo Nobel N.V.**
**6824 BM Arnhem (NL)**

(72) Inventor:
**Gingeras, Thomas Raymond**
**Encinitas, California 92024 (US)**

(74) Representative:
**Van Gent, Marieke et al**
**N.V. Organon**
**Patent Department Pharma**
**Weth v. Eschstraat 1**
**P.O. Box 20**
**5340 BH Oss (NL)**

(56) References cited:
**EP-A- 0 200 362**

• **NUCLEIC ACIDS RESEARCH. vol. 12, no. 18 , 25 September 1984 , ARLINGTON, VIRGINIA US pages 7035 - 56 MELTON, D. ET AL 'Efficient in vitro synthesis of biologically active RNA and RNA hybridization probes from plasmids containing a bacteriophage SP6 promoter'**

• **FEBS LETTERS. vol. 212, no. 2 , February 1987 , AMSTERDAM NL pages 271 - 275 KRUPP, G. ET AL. 'Simplified in vitro synthesis of mutated RNA molecules.'**

• **SCIENCE. vol. 230 , 20 December 1985 , LANCASTER, PA US pages 1350 - 55 SAIKI, R. ET AL 'Enzymatic amplification of beta-globin genomic sequences and restriction site analysis for diagnosis of sickle cell anemia'**

• **NUCLEIC ACIDS RESEARCH. vol. 14, no. 14 , 1986 , ARLINGTON, VIRGINIA US pages 5591 - 5603 CHU, F ET AL. 'SYNTHESIS OF AMPLIFIABLE REPORTER RNA FOR BIOASSAYS'**

• **SCIENCE. vol. 239 , 29 January 1988 , LANCASTER, PA US pages 491 - 493 STOFLET, E. ET AL. 'Genomic amplification with transcript sequencing'**

• **NUCLEIC ACIDS RESEARCH. vol. 15, no. 21 , October 1987 , ARLINGTON, VIRGINIA US pages 8783 - 98 MILLIGAN, J. ET AL 'Oligonucleotide synthesis using T7 RNA polymerase and synthetic DNA templates'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Field of the Invention

The present invention relates generally to advances in molecular biology and molecular genetics.

More particularly, the present invention relates to novel methods containing requisite reagents and means for increasing the in vitro or x-vivo copy number of, i.e., amplifying, at least one selected segment (sequence) of nucleic acid or its complement or hybridizing homologous segment, in a sample comprising one or more nucleic acids, which may include RNA, DNA or both.

Among the applications in which the methods of this invention find utility are 1) in analyses of body fluids and tissues for the detection of specific nucleic acid sequences characteristic of a genetic or pathogenic disease or condition by in vitro or x-vivo nucleic acid probe hybridization assays and 2) in the selective cloning of single-copy or rare or low-expression genes.

Background of the Invention

Much of the work in molecular biology, molecular genetics and applications thereof, such as for example, nucleic acid probe hybridization assays for blood-borne pathogens or defective genes, involves the detection or isolation of a particular nucleic acid sequence. A fundamental problem in such work is to detect or isolate and then quantitate a nucleic acid sequence of interest. The problem has been a difficult one because biological materials, such as cell cultures, tissue specimens, and blood samples, typically are comprised of a complex mixture of RNA and DNA entities of which at most only a minuscule fraction has a sequence of interest.

Indeed, practical applications of nucleic acid probe hybridization assays have been limited because the sensitivity of the assays, when carried out with reagents suitable for routine use and over acceptably short time periods, is too low to detect sequences of interest at the low concentration at which they occur in real samples.

Two fundamentally different approaches have been taken to address the problem of detecting a nucleic acid sequence of interest ("target segment") present at a low level in a complex mixture of nucleic acids.

In the first approach, the amount of nucleic acid (including the target segment) in a sample is not altered; instead, a signal-generating system is associated with the target segment and produces a detectable signal representative of the number of molecules of target segment. For example, a nucleic acid probe, with a sequence complementary to that of a subsegment of the target segment is linked to an enzyme such as alkaline phosphatase and is mixed with sample under hybridization conditions that will effect hybridization between the probe and the target segment (but not appreciably between probe and other nucleic acid sequences in the sample). After removing enzyme-linked probe that failed to hybridize, a chromogenic substrate for alkaline phosphatase is added under suitable conditions and, in principle, a large number of detectable, colored molecules is rapidly produced for each probe molecule hybridized to target segment.

Numerous other systems for detecting nucleic acid sequences without altering the amount of target nucleic acid in the sample are known to the art. Another example is the commonplace labeling of a nucleic acid probe with radioactive atoms such as $^{32}$P and then detecting probe hybridized to target via amplified signal initiated by decay of the radioactive nuclei. Still another example involves linking to probe for the target segment another nucleic acid that is capable of replication such that it can be readily detected by known techniques. Certain RNAs are known that are susceptible to (autocatalytically) replicase-induced replication by certain polymerase, such as bacteriophage RNA-dependent RNA polymerase, such as Qβ replicase and the replicase from brome mosaic virus (BMV). In such a system, both an RNA and the RNA of complementary sequence are templates for replication by the RNA polymerase; consequently the amount of replicated RNA increases exponentially with time (as long as the number of RNA template molecules does not exceed the number of RNA polymerase molecules in a system). See Miele et al., J. Mol. Biol. 171, 281 (1983). A system in which probe for a target segment is linked to an RNA capable of being replicated by Qβ replicase is described by Chu et al., Nucl. Acids Res. 14, 5591 (1986) and that by BMV replicase by Marsh et al., Positive strand RNA Viruses, Alan R. Liss (publr.; New York) (1987; Proceedings of UCLA Symposium, 1986).

The first approach has two serious drawbacks. First, in many instances, the copy number of target segment in a sample of practical size is so low that, even for reasonably rapid signal generating systems, the time required to generate detectable signal that is significantly above background is impractically long. Second, signal generation occurs at essentially the same rate from "background" signal generating molecules as from signal generating molecules associated with target. In any assay for a target segment, a signal due to "background" is unavoidable; that is, invariably there is some signal due to probe that non-specifically adheres to filters or other solid supports or hybridizes to segments with sequences closely similar to that of target segment. If the copy number of target is too low, the time constant ratio of signal from target plus background (i.e. "signal") to signal from background (i.e. "noise") will be too low to be detectable significantly above background. These and other drawbacks have led the art to a second approach of addressing the problem of detecting a target segment present at a low level in a complex mixture of nucleic acids.

This second approach is fundamentally different and involves increasing the copy number of the target segment itself, preferably to an extent greater than that of other sequences in a sample, particularly those that might erroneously be detected as target segment because of similarities in sequence. Examples of this second approach include various culture techniques in which cells that harbor the target segment are caused to increase in number, sometimes more rapidly than numbers of other cells, or in which particular nucleic acids (e.g., plasmids, RNAs) therein having disposed target segment are caused to increase in number.

Such culture techniques have the disadvantages of being cumbersome and problematic and time-consuming and manifest the inevitable: nucleic acids other than those which include target segment are simultaneously increased in copy number, thus potentially increasing "background." Another disadvantage is the resultant growth of potentially dangerous organisms as a necessary step to achieve amplification.

Another example of this second approach is amplification of a DNA target segment in a so-called "polymerase chain reaction" ("PCR"). This technique is a borrowed adaptation of known, naturally occurring processes occurring in the replicative process of, for example, single-stranded DNA genomes of certain virus entities, and in all events, represents an application akin to cDNA preparation ala Hong, Bioscience Reports 1, 243 (1981); Cooke et al., J. Biol. Chem. 255, 6502 (1980); and Zoller et al., Methods in Enzymology 100, 468-500 (1983). By this technique, a particular segment increases in copy number exponentially with a number of cycles, each of which entails (1) hybridizing to a 3'-terminal subsegment of each of the target segment and its complement (i.e., the segment of sequence complementary to that of target segment) a DNA primer, (2) extending each of the primers with a DNA polymerase, and (3) rendering single stranded by thermal denaturation the duplexes resulting from step (2). This technique is described in Saiki et al., Science 230, 1350 (1985), and Mullis et al., European Patent Application Publication Nos. 200362 and 201184. See also U.S. Patents 4683195 and 4683202. Reportedly, in applying the technique for 20 cycles over about 3 hours, the copy number of a target segment can be increased by a factor of about $10^5$. Because only those segments to which a specific primer hybridizes with a sufficient stability to initiate chain extension by the polymerase to form the complement and which have a complement to which another specific primer hybridizes similarly to yield target segment upon chain extension, they increase exponentially in copy number while other non-target segments not erroneously hybridized with the employed primer increase, if at all, at most linearly in copy number as a function of number of cycles. The polymerase chain reaction technique can greatly increase not only the copy number of target segment but also the ratio of the amount of target segment to that of background-causing segments in a sample.

Of course, it follows that this second approach can be applied to a sample in conjunction with use of the first approach applied with the amplified target segment to provide even a stronger detection signal.

A further development of the second approach is described in EP-A-0310229 which forms part of the state of the art by virtue of Article 54(3)EPC. The method involves generating a double stranded DNA intermediate comprising a promotor followed by a target sequence. This intermediate is then used to prepare multiple RNA copies using an RNA polymerase.

It is an object of the present invention to solve the problems addressed by the prior art and to overcome the disadvantages enumerated in prior researchers endeavors to solve those problems. It is a further object of the present invention to provide a straightforward technique that can be utilized in an acceptably short time, employing the convenience of known reagents and having the precision necessary to reach consistent scientific results; one that can be employed in a reproducible assay setting and that is adaptable for use in kits for laboratory/clinical analyses.

It is thus an object of the present invention to increase the detectability of certain nucleic acid sequences (target segments) by amplification of said target sequences in an in vitro or x-vivo system devoid of the disadvantages enumerated thus far by prior art endeavors.

The present invention is concerned with a novel technique for carrying out the second approach to detecting a target segment present at a low level in a complex mixture of nucleic acids. It employs a novel RNA transcript production step in conjunction with, and derived from, a synthesized double-stranded cDNA copy of the target sequence as a complete cycle. Multiple cycles can be employed. By virtue of the transcription step being the dominant aspect of novelty, it is conveniently referred to herein as a transcription-based amplification system (TAS). The novel technique of the present TAS invention results in rapid increase in copy number of a selected target segment by making use of two properties of DNA-dependent RNA polymerase: (1) appreciable initiation of transcription from only a small number of sequences specific for each polymerase, see, e.g., Brown et al., Nucl. Acids Res. 14, 3521 (1986); and (2) rapid production of a large number of transcripts from each copy of a promoter (typically $10^2$-$10^4$ per hour) recognized by an RNA polymerase. See Milligan et al., Nucleic Acids Res. 15, 8783 (1987). The technique of the invention can also utilize the ability of RNAs with certain sequences to be rapidly (autocatalytically) replicated by RNA-dependent RNA replicases. See also Miele et al., supra. In addition, it provides a standardization technique making possible unambiguous measurement of the amount of target DNA present in a sample.

The present invention (unless induced (autocatalytic) replication is employed) yields a single-stranded RNA transcript, or an RNA-DNA duplex formed therefrom when measures are not undertaken to prevent its formation, that has a subsegment that has the sequence of the target segment or the sequence complementary to that of the target segment, and that is present in large excess relative to nucleic acid with a subsegment of complementary sequence. This excess initially of a single-stranded RNA transcript is advantageous in certain methods for detecting amplified product with a labeled nucleic acid probe because little segment of complementary sequence is present to compete with probe for hybridizing to the amplified product. Also, the single-stranded RNA transcript product hereof is struck-off more or less continuously and provides for direct detection of target segment without the necessity of cumbersome, error-prone repeated PCR cycles and strand separation. Such advantages are not provided by the PCR technique that yields double-stranded DNA (one strand of which comprises target segment and the other strand of which comprises complement of target segment) that need to be separated before detection and only after a large number of repeated cycles necessary to reach acceptable amplification levels.

The techniques of the present invention provide amplification of a selected target segment to an extent at least as great as the PCR technique over about the same period of time, but in a far more simplistic and reproducible manner and distinct from natural processes or other art.

Summary of the Invention

In particular, in accordance with the invention there is provided a method for amplifying a target nucleic acid sequence in a sample containing nucleic acid comprising:

(1) hybridizing to said target nucleic acid sequence a first single-stranded DNA primer, wherein said primer comprises a promoter sequence linked by a sequence of 0 to 100 nucleotides to a sequence of nucleotides which are complementary to the 3' end of said target sequence, said complementary sequence being at least 10 nucleotides in length,

(2) extending said first primer hybridized in accordance with step (1) in a reaction catalysed by a DNA polymerase to form a first complementary DNA sequence,

(3) rendering single-stranded the duplex formed in step (2),

(4) hybridizing to said first complementary DNA sequence a second single-stranded DNA primer having a sequence of at least 10 nucleotides corresponding to the 5' end of said target sequence and optionally a further DNA sequence of up to 100 nucleotides,

(5) extending said second primer hybridized in accordance with step (4) in a reaction catalysed by a DNA polymerase to form a second complementary DNA sequence and

(6) employing the double-stranded DNA product of step (5) in the preparation of a plurality of RNA transcripts complementary to said target sequence in a reaction catalysed by a DNA-dependent RNA polymerase with specificity for the promoter comprised in said first primer.

We have discovered how bacteriophage DNA-dependent RNA polymerase can be used to rapidly amplify (i.e., increase the copy number of) a selected target nucleic acid sequence (target sequence or segment) present in a sample of nucleic acids. Further, we have discovered how bacteriophage DNA-dependent RNA polymerase can be used together with bacteriophage RNA-dependent RNA polymerase to accomplish the same result. Heretofore, it has not been appreciated that such bacteriophage RNA polymerase could be used for this purpose.

The invention entails methods based on these discoveries.

These methods are particularly usefully applied in connection with nucleic acid probe hybridization assays for a nucleic acid which includes a target segment amplified in accordance with the invention. Thus, the invention also entails methods, and kits for carrying out the methods, for detecting the presence or absence of a segment in a sample of a nucleic acid, which comprises a particular segment, by means of a probe hybridized to that segment after amplification in accordance with the invention.

The present invention is predicated on the novelty of certain RNA transcripts, their production, optional replication, and use, to achieve desired amplification and detection of corresponding (in sequence) target nucleic acid sequence. The invention is practiced in an in vitro or x-vivo setting, and is employed conjunctively with the synthesis of a double-stranded cDNA copy of the target sequence in order to produce a double-stranded nucleic acid template used in turn for production of said RNA transcripts. This process of double-stranded cDNA synthesis and RNA transcription constitutes a single cycle of the present transcription-based amplification system (TAS). If desired, this cycle may be repeated in order to achieve even higher levels of amplification. By virtue of the method by which they are produced (and reproduced), the transcripts correspond (identically or complementarily) in sequence to a target nucleic acid sequence contained in an original sample amongst a mixture of nucleic acids, and therefore, the presence of the transcripts in amplified form provides for their detection, and hence by correspondence, the in vitro or x-vivo detection of the presence of the target nucleic acid sequence in said sample.

Thus, the present invention involves the in vitro or x-vivo detection of at least one specific nucleic acid sequence (target sequence or segment) in a sample containing nucleic acid. The present invention reduces to a method comprising preparing a double-stranded nucleic acid containing a sequence corresponding to a target sequence operably linked to a promoter therefor, employing said double-stranded nucleic acid as a double-stranded nucleic acid template for the preparation of a plurality of RNA transcripts therefrom, each bearing an RNA sequence corresponding to said target sequence, and detecting the presence of said RNA sequence and by analogy the presence of target sequence.

The present invention is directed to all methods and means associated with the preparation and use of such RNA transcripts, for example the optionally repetitive method of preparing said double-stranded nucleic acid template defined above can be used comprising providing a first nucleic acid primer containing a promoter sequence operably linked to a sequence corresponding to a segment of a target sequence, as defined above, hybridizing under suitable conditions said first nucleic acid primer with target sequence in a sample containing nucleic acid, extending the hybridized said first nucleic acid primer in a polymerase extension reaction complementarily to the target sequence to form a corresponding duplex nucleic acid, separating the strands of said duplex, hybridizing to the separated promoter containing sequence strand under suitable conditions a second nucleic acid primer at the end opposite said promoter sequence, and extending the hybridized said second nucleic acid primer in a polymerase extension reaction complementarily to said promoter containing sequence.

Further, and alternative, methods and means of preparing said double-stranded nucleic acid template (supra) are, for example, an essentially single-pot reaction comprising providing a first nucleic acid primer containing a promoter sequence operably linked to a sequence complementary to a segment of a target sequence and a second nucleic acid primer having a sequence identical to a segment of a target sequence, said primers corresponding to different regions of said target sequence but not, or not substantially, overlapping in their correspondence to the target and being selected such that an extension product of one when separated from its complement can serve as a template for an extension product of the other, contacting a sample containing nucleic acid including target sequence with said primers under sequential hybridizing and strand separation conditions so as to produce, in turn, extension products of said primers.

The present invention is directed to methods and means of employing said double-stranded nucleic acid supra., as a template for the preparation of a plurality of RNA transcripts therefrom in a reaction catalyzed by a DNA-dependent RNA polymerase that recognizes the promoter thereof, and detecting and measuring, the presence of said RNA transcripts.

The present invention is further directed to methods and means to standardize the amount of target sequence detected (by correspondence to the amount of RNA transcript detected) by further correlation to the presence of known nucleic acid used as an internal standard. The copy number of the standard is predetermined, the standard shall experience the same conditions, hence fate, during the practice of this invention as does target sequence and it shall therefore serve as an evaluation means in determining relative amounts of transcripts prepared in parallel for it and for target sequence.

The present invention is further directed to the further replication of obtained RNA transcripts defined above via the presence therein of replicase recognition sites. This is conveniently accomplished by providing said first nucleic acid primer defined above additionally bearing a replicase recognition site sequence, preferably between the promoter sequence and sequence corresponding to target sequence, and/or additionally containing a replicase recognition site on the second nucleic acid primer defined above. On subsequent transcription, the consequent transcripts bear the replicase recognition site(s), such that presence of a replicase (in the reaction locus or kit for example) (autocatalytically) induces replication of the transcripts producing additional copies to further facilitate detection.

Detailed Description of the Invention

1. Brief Description of the Drawings

Figures 1A, 1B and 1C illustrate a method according to the invention for amplifying a target segment of a nucleic acid (Nucleic Acid A), which is a DNA or RNA, wherein many copies of a first RNA (RNA I) with a segment with a sequence complementary to that of target segment are made.

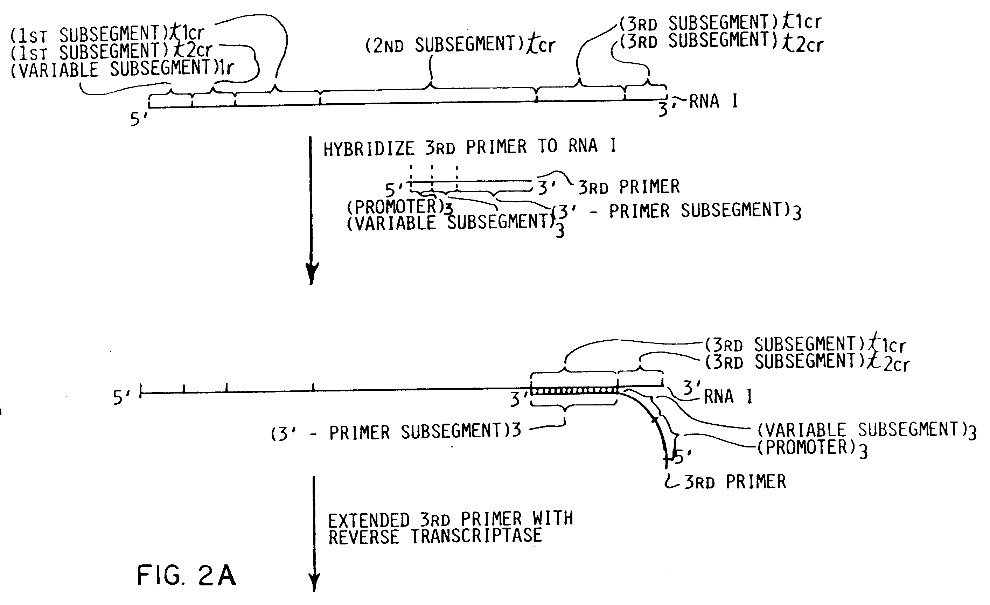

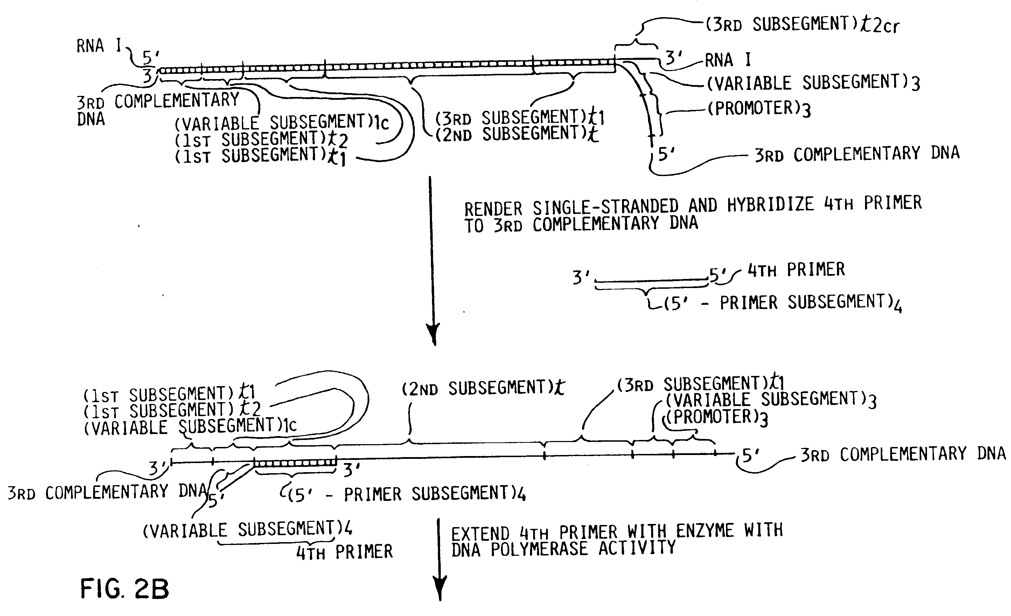

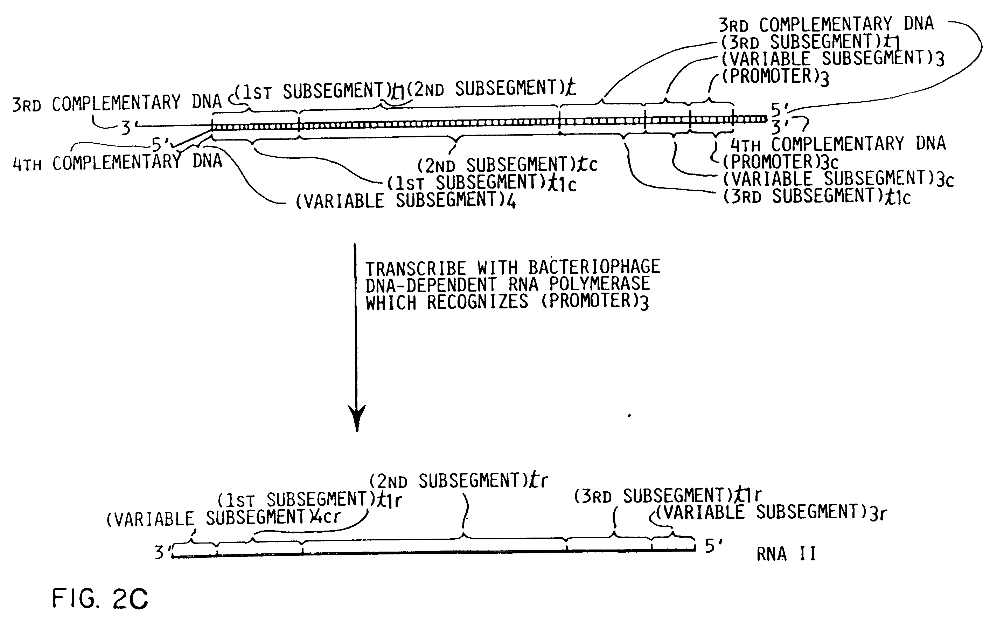

Figures 2A, 2B, 2C illustrate the further amplification according to the invention of a segment of an RNA I, made as illustrated in Figures 1A, 1B and 1C, to make many copies of a second RNA (RNA II) with a segment with a sequence the same as that of a subsegment of the target segment, which was amplified to make the RNA I.

Figure 3 depicts autoradiograms showing various concentrations of HIV RNA amplified by TAS simultaneously with a fixed concentration of human β-globin nucleic acid.

Figure 4 displays a general strategy whereby RNA produced through a DNA-dependent RNA polymerase yields an RNA molecule that can serve as a template for an RNA-dependent replicase.

2. General Methods and Definitions

Reference is made to standard textbooks of molecular biology that contain definitions and methods and means for carrying out basic techniques of the present invention such as: DNA probe or primer preparation, including DNA synthesis; hybridization methodology including variations in stringency conditions for producing more or less hybridization certainty depending upon the degree of homology of the primer to a target DNA sequence; identification, isolation or preparation of promoters, or more specifically promoters or sites recognized by bacteriophage DNA-dependent RNA polymerase and bacteriophage RNA-dependent RNA polymerase, or in the employment of eukaryotic systems, viral DNA- and RNA-dependent RNA polymerases, for example, adenovirus encoded RNA polymerase and brome mosaic virus RNA polymerase; conditions conducive to the production of RNA transcripts, including so-called transcription enhancer sequences; the mechanism and methodology for (induced) replication; polymerase chain reaction methods including the reagents used therein; and so forth. See, for example, Maniatis et al., Molecular Cloning: A Laboratory Manual, Cold Spring Harbor Laboratory, New York, (1982), and the various references cited therein; U.S. Patent 4683195; U.S. Patent 4683202; Hong, Bioscience Reports 1, 243 (1981); Cooke et al., J. Biol. Chem. 255 6502 (1980); and Zoller et al., Methods in Enzymology 100, 468-500 (1983); Crea et al., Nucleic Acids Res. 8, 2331 (1980); Narang et al., Meth. Enzym. 68, 90 (1979); Beaucage et al., Tetrahedron Letters 22, 1859 (1981); Brown et al., Meth. Enzym. 68, 109 (1979); Caruthers et al., Meth. Enzym. 154, 287 (1985); Hitzeman et al., J. Biol. Chem. 255, 2073 (1980); Lee et al., Science 239, 1288 (1988); Milligan et al., Nucleic Acids Res. 15, 8783 (1987); Miller et al., Virology 125, 236 (1983), Ahlquist et al., J. Mol. Biol. 153, 23 (1981); Miller et al., Nature 313, 68 (1985); Ahlquist et al., J. Mol. Biol. 172, 369 (1984); Ahlquist et al., Plant Mol. Biol. 3, 37 (1984); Ou et al., PNAS 79, 5235 (1982); Chu et al., Nucl. Acids Res. 14, 5591 (1986); European Patent Application Publn. No. (EPA) 194809; Marsh et al., Positive Strand RNA Viruses, p. 327-336, Alan R. Liss (publ.; New York) (1987; Proceedings of UCLA Symposium, 1986); Miller et al., J. Mol. Biol. 187, 537 (1986); Stoflet et al., Science 239, 491 (1988); and Murakawa et al., DNA 7, 287 (1988).

By the term "promoter" is meant a nucleic acid sequence (naturally occurring or synthetically produced or a product of restriction digest) that is specifically recognized by an RNA polymerase that binds to a recognized sequence and initiates the process of transcription whereby an RNA transcript is produced. It may optionally contain nucleotide bases extending beyond the actual recognition site, thought to impart additional stability toward degradation processes. In principle, any promoter sequence may be employed for which there is a known and available polymerase that is capable of recognizing the initiation sequence. Typical, known and useful promoters are those that are recognized by certain bacteriophage polymerase such as bacteriophage T3, T7 or SP6. See Siebenlist et al., Cell 20, 269 (1980). These are but examples of those polymerase which can be employed in the practice of the present invention in conjunction with their associated promoter sequences.

The "RNA transcript" hereof is the ribonucleic acid sequence produced after transcription initiation following RNA polymerase recognition of the promoter sequence (See supra). The production of such transcripts is more or less continuous, dependent in part on the amount of polymerase present.

By the term "primer" in the present context is meant a nucleic acid sequence (naturally occurring or synthetically produced or a product of restriction digest) that has sufficient homology with the target sequence such that under suitable hybridization conditions it is capable of hybridizing, that is binding to, the target sequence. A typical primer is at least about 10 nucleotides in length, and most preferably is of approximately 35 or more nucleotide bases in length, and in its most preferred embodiments, it shares identity or very high homology with the target sequence. See, for example, EPA 128042 (publd. 12 Dec 84).

The term "operably linked" in particular in connection with the linkage of a promoter sequence within a primer sequence, refers to the functionality of the ultimate "double-stranded nucleic acid template" of the present invention such that it, the template, is capable of producing corresponding RNA transcripts when the promoter is recognized by the suitable polymerase--see supra.

The primer extension reaction to produce a duplex is known per se. See references supra. Polymerase useful for this purpose include E. coli DNA polymerase I, Klenow fragment of E. coli DNA polymerase I, T4 DNA polymerase, reverse transcriptase, and so forth.

The techniques of forming a detection signal such as via radioactive labeling or chromogenic means using a chromogenic susceptible enzyme are also well known and documented in the art. See discussion supra.

The use of a "replicase" for (autocatalytic) induction of replication of the RNA transcripts of the present invention are generally known in the art. Suitable examples of such replicases that are useful in the present invention include the so-called Qβ virus replicase that recognizes certain nucleic acid sequence sites at both the 3'- and 5'- ends of the given RNA transcript and the so-called brome mosaic virus (BMV) as well as the alpha virus replicases which are thought to recognize nucleic acid sequence sites at the 3'- end of a given RNA transcript. These replicases serve to replicate, that is reproduce, the RNA transcripts and complements so as to multiply copies thereof. When such enzyme is present in the reaction locus during the process of transcription, it can be foreseen that the multiple transcripts that are produced during transcription can themselves undergo replication so as to exponentially increase the amount of RNA transcript product.

Internal standardization is defined as a process which is used to: a) insure that the TAS amplification process has not failed because of a procedural error, and b) measure the levels of target nucleic acid relative to a predetermined quantity of nucleic acid which is always associated with the sample of interest. Such internal standardization occurs by coamplifying a portion of the target sequence, as well as an endogenous sequence, in the same reaction. For example, by knowing the cell count present within the biological sample, a single-copy gene (e.g., β-globin) could be used as an internal standard and, since it is not expressed in the form of RNA, its initial copy number is equal to two times the total number of cells in the sample. By coamplifying portions of the β-globin and target sequences of interest, the ratios of the amplified signals can be compared to quantitate the levels of target sequence of interest. Since every cell has two copies (in diploid cells) of this internal standard, irrespective of whether the biological sample contains separate target sequences (e.g., HIV), each sample is expected to produce a positive amplification signal resulting from the internal standard. See Groudine et al., Nucleic Acids Research 12, 1427 (1984) and McKnight, Cell 31, 355 (1982). See also British Patent Application Publn. No. 2187283 A (publd. 3 Sept. 87).

3. Detailed Description of Preferred Aspects

In one of its aspects, the invention is a method for amplifying a target nucleic acid segment of Formula I

3'-(first subsegment)$_t$-(second subsegment)$_t$-(third subsegment)$_t$-5'

I

wherein (first subsegment)$_t$ is a nucleic acid segment of known sequence of at least 10 nucleotides adjoining the 3'-terminus of (second subsegment)$_t$, (second subsegment)$_t$ is a nucleic acid segment of 0 or more nucleotides, and (third subsegment)$_t$ is a nucleic acid segment of known sequence of at least 10 nucleotides adjoining the 5'-terminus of (second subsegment)$_t$, which method comprises:

(1) hybridizing to (first subsegment)$_t$ of said target segment a first primer, which is a single-stranded DNA which comprises a 3'-terminal subsegment of Formula II

5'-(promoter)$_1$-(variable subsegment)$_1$-(3'-primer subsegment)$_1$-3'

II

wherein (promoter)$_1$ is a single-stranded DNA segment with the sequence of the plus-strand of a bacteriophage DNA-dependent RNA polymerase-specific promoter, (variable subsegment)$_1$ is a single-stranded DNA segment of 0 to 100 nucleotides adjoining the 3'-terminal nucleotide of (promoter)$_1$ and the 5'-terminal nucleotide of (3'-primer subsegment)$_1$, and (3'-primer subsegment)$_1$ is a single-stranded DNA segment of at least 10 nucleotides with a sequence which is complementary to the sequence of a subsegment of (first subsegment)$_t$ which terminates with the 3'-terminal nucleotide of (first subsegment)$_t$, said (promoter)$_1$ adjoining the 5'-terminus of said (3'-primer subsegment)$_1$, if said (variable subsegment)$_1$ has 0 nucleotides;

(2) extending said first primer, hybridized in accordance with step (1), in a reaction catalyzed by a first DNA polymerase to make a first complementary DNA segment, which comprises a subsegment of Formula III

5'-(promoter)$_1$-(variable subsegment)$_1$-(first subsegment)$_{tc}$-(second subsegment)$_{tc}$-(third subsegment)$_{tc}$-3',

III

wherein (first subsegment)$_{tc}$ is the DNA segment with the sequence complementary to that of (first subsegment)$_t$, (second subsegment)$_{tc}$ is the DNA segment with the sequence complementary to that of (second subsegment)$_t$, and (third subsegment)$_{tc}$ is the DNA segment with the sequence complementary to that of (third subsegment)$_t$, provided that, if the target segment is an RNA segment, said first DNA polymerase is a reverse transcriptase;

(3) rendering single-stranded the duplex formed in the reaction of step (2);

(4) hybridizing to (third subsegment)$_{tc}$ of said first complementary DNA of Formula III a second primer, which is a single-stranded DNA of at least 10 nucleotides of Formula IV 3'-(5'-primer subsegment)$_2$-(variable subsegment)$_2$-5',

IV

wherein (5'-primer subsegment)$_2$ has the sequence of a subsegment of (third subsegment)$_t$ which terminates with the 5'-terminal nucleotide of (third subsegment)$_t$ and wherein (variable subsegment)$_2$ is a segment of 0 to 100 nucleotides which adjoins the 5'-terminus of (5'-primer subsegment)$_2$;

(5) extending said second primer segment, hybridized in accordance with step (4), in a reaction catalyzed by a second DNA polymerase to form a second complementary DNA segment which comprises a subsegment of Formula V

5'-(variable subsegment)$_2$-(third subsegment)$_t$-(second subsegment)$_t$-(first subsegment)$_t$-(variable subsegment)$_{1c}$-(promoter)$_{1c}$-3',
        V

wherein (variable subsegment)$_{1c}$ is the DNA segment with the sequence complementary to that of (variable subsegment)$_1$, and (promoter)$_{1c}$ is the DNA segment with the sequence complementary to that of (promoter)$_1$, provided that said second DNA polymerase is the same as or different from said first DNA polymerase; and

(6) employing the double-stranded product of step (5) as the template in a reaction catalyzed by a first bacteriophage DNA-dependent RNA polymerase, which recognizes the promoter of which one strand is (promoter)$_1$, to make a first RNA product of Formula VI

5'-(variable subsegment)$_{1r}$-(first subsegment)$_{tcr}$-(second subsegment)$_{tcr}$-(third subsegment)$_{tcr}$-(variable subsegment)$_{2cr}$-3'
        VI

wherein (variable subsegment)$_{1r}$ is the RNA segment with the sequence of (variable subsegment)$_1$, (first subsegment)$_{tcr}$ is the RNA segment with the sequence complementary to that of (first subsegment)$_t$, (second subsegment)$_{tcr}$ is the RNA segment with the sequence complementary to that of (second subsegment)$_t$, (third subsegment)$_{tcr}$ is the RNA segment with the sequence complementary to that of (third subsegment)$_t$, and (variable subsegment)$_{2cr}$ is the RNA segment with the sequence complementary to that of (variable subsegment)$_2$.

In another of its aspects, the invention entails a method wherein (first subsegment)$_t$ is at least 10 nucleotides in length and is of Formula XIII

3'-(first subsegment)$_{t2}$-(first subsegment)$_{t1}$-5',
       XIII

wherein (first subsegment)$_{t2}$ has 0 or more nucleotides and, if more than 0, adjoins the 3'-terminus of (first subsegment)$_{t1}$, and (first subsegment)$_{t1}$ is at least 10 nucleotides in length; wherein (third subsegment)$_t$ is of Formula XIV

3'-(third subsegment)$_{t1}$-(third subsegment)$_{t2}$-5,
        XIV

wherein (third subsegment)$_{t2}$ has 0 or more nucleotides and, if more than 0, adjoins the 5'-terminus of (third subsegment)$_{t1}$, and (third subsegment)$_{t1}$ is at least 10 nucleotides in length; wherein (3'-primer subsegment)$_1$ has the sequence complementary to that of a subsegment of target segment which consists of all of (first subsegment)$_{t2}$ and 0 or more nucleotides of (first subsegment)$_{t1}$; wherein (5'-primer subsegment)$_2$ is a subsegment which consists of all of (third subsegment)$_{t2}$ and 0 or more nucleotides of (third subsegment)$_{t1}$; and wherein, after steps (1) to (6) Supra, the RNA subsegment of Formula VII

5'-(first subsegment)$_{t1cr}$-(second subsegment)$_{tcr}$-(third subsegment)$_{t1cr}$-3'
       VII

wherein (first subsegment)$_{t1cr}$ is the RNA segment with the sequence complementary to that of (first subsegment)$_{t1}$ and (third subsegment)$_{t1cr}$ is the RNA segment with the sequence complementary to that of (third subsegment)$_{t1}$, is further amplified by a method which comprises:

(7) hybridizing to said first RNA product of Formula VI a third primer, which is a single-stranded DNA which comprises a 3'-terminal subsegment of Formula VIII

5'-(promoter)$_3$-(variable subsegment)$_3$-(3'-primer subsegment)$_3$-3',
       VIII

wherein (promoter)$_3$ is a single-stranded DNA segment with the sequence of the plus-strand of a bacteriophage DNA-dependent RNA polymerase-specific promoter, said sequence of (promoter)$_3$ being the same as or different from that of (promoter)$_1$, (variable subsegment)$_3$ is a single-stranded DNA segment of 0 to 100 nucleotides which adjoins the 3'-terminal nucleotide of (promoter)$_3$ and the 5'-terminal nucleotide of (3'-primer subsegment)$_3$, and (3'-primer subsegment)$_3$ is a single-stranded DNA segment which has the same sequence as (third subsegment)$_{t1}$ and adjoins the 3'-terminal nucleotide of (promoter)$_3$ if (variable subsegment)$_3$ has 0 nucleotides;

(8) extending said third primer hybridized in accordance with step (7) in a reaction catalyzed by a third DNA polymerase, which is a reverse transcriptase and is the same as or different from said first and second DNA polymerase, to make a third complementary DNA segment which comprises a 3'-terminal subsegment of Formula IX

5'-(promoter)$_3$-(variable subsegment)$_3$-(third subsegment)$_{t1}$-(second subsegment)$_t$-(first subsegment)$_{t1}$-(first subsegment)$_{t2}$-(variable subsegment)$_{1c}$-3',

IX

wherein (variable subsegment)$_{1c}$ is the DNA with the sequence complementary to that of (variable subsegment)$_1$;

(9) rendering single-stranded the duplex formed in the reaction of step (8);

(10) hybridizing to the third complementary DNA made in the reaction of step (8) a fourth primer of Formula X
5'-(variable subsegment)$_4$-(5'-primer subsegment)$_4$

X

wherein (variable subsegment)$_4$ is a segment of 0 to 100 nucleotides, and (5'-primer subsegment)$_4$ is a subsegment of known sequence which adjoins the 3'-nucleotide of (variable subsegment)$_4$, if (variable subsegment)$_4$ has more than 0 nucleotides, and which comprises at least 10 nucleotides of the segment of Formula XX
5'-(variable subsegment)$_1$-(first subsegment)$_{t2c}$-(first subsegment)$_{t1c}$-3',

XX

wherein (first subsegment)$_{t2c}$ is the DNA segment with the sequence complementary to that of (first subsegment)$_{t2}$ and (first subsegment)$_{t1c}$ is the DNA segment with the sequence complementary to that of (first subsegment)$_{t1}$, provided that at least one of said at least 10 nucleotides is at or 5' from the 5'-terminal nucleotide of (first subsegment)$_{t1c}$;

(11) extending said fourth primer hybridized in accordance with step (10) in a reaction catalyzed by a fourth DNA polymerase, which is the same as or different from said first, second and third DNA polymerase, to form a fourth complementary DNA which comprises a segment of Formula XI
5'-(first subsegment)$_{t1c}$-(second segment)$_{tc}$-(third subsegment)$_{t1c}$-(variable subsegment)$_{3c}$-(promoter)$_{3c}$-3',

XI

wherein (second subsegment)$_{tc}$ is the DNA segment with the sequence complementary to that of (second subsegment)$_t$, (third subsegment)$_{t1c}$ is the DNA segment with the sequence complementary to that of (third subsegment)$_{t1}$, (variable subsegment)$_{3c}$ is the DNA segment with the sequence complementary to that of (variable segment)$_3$, and (promoter)$_{3c}$ is the DNA segment with the sequence complementary to that of (promoter)$_3$; and

(12) employing the double-stranded product of step (11) as the template in a reaction catalyzed by a second bacteriophage DNA-dependent RNA polymerase, which is the same as or different from said first bacteriophage DNA-dependent RNA polymerase and which recognizes the promoter of which one strand is (promoter)$_3$, to make a second RNA product with a 5'-terminal subsegment of Formula XII 5'-(variable subsegment)$_{3r}$-(third subsegment)$_{t1r}$-(second subsegment)$_{tr}$-(first subsegment)$_{t1r}$-X$_{12}$-(variable subsegment)$_{4cr}$-3',

XII

wherein (variable subsegment)$_{3r}$ is the RNA segment with the sequence of (variable subsegment)$_3$, (third subsegment)$_{t1r}$ is the RNA segment with the sequence of (third subsegment)$_{t1}$, (second subsegment)$_{tr}$ is the RNA segment with the sequence of (second subsegment)$_t$, (first subsegment)$_{t1r}$ is the RNA segment with the sequence of (first subsegment)$_{t1}$, (variable subsegment)$_{4cr}$ is the RNA segment with the sequence complementary to that of (variable subsegment)$_4$, and X$_{12}$ is the RNA segment with the sequence complementary to that of the subsegment of (5'-primer subsegment)$_4$ that is 5' from the 5'-terminus of (first subsegment)$_{t1c}$.

Methods according to the invention for carrying out nucleic acid hybridization probe assays which entail amplifying a target segment in accordance with the invention entail, in addition to the steps and components, respectively, of the methods for amplification, steps necessary for detecting the RNA product resulting from amplification according to the invention. The skilled understand the various additional steps, respectively, that are required to detect RNA from an amplification process by any of the numerous nucleic acid probe hybridization assay methods known in the art. One preferred nucleic acid probe hybridization assay method, involving bead-capture of labeled RNA amplification, is illustrated in Example II below.

It is preferred that (3'-primer subsegment)$_1$, (5'-primer subsegment)$_2$, (3'-primer subsegment)$_3$, and (5'-primer subsegment)$_4$ have between 20 and 40 nucleotides, and more preferably about 30 nucleotides to enhance the specificity with which the various primers bind to the ends of the target segments which are sought to be amplified.

It is also preferred that the target segment of a nucleic acid of interest which is selected for amplification (by virtue of selection of (first subsegment)t and (third subsegment)t be selected so that (second subsegment)$_t$r have at

least 30, and more preferably at least about 50, nucleotides. This permits the use of (second subsegment)$_{tcr}$ or (second subsegment)$_{tr}$ in the amplified RNA product to be targets for nucleic acid probes that will not have sequences that overlap those of any of the primer subsegments.

While the bacteriophage DNA-dependent RNA polymerase can use templates longer than 1000 bp, their efficiency of transcription decreases as the template becomes longer. Thus, target segments less than 1000 bases long are favored.

It is preferred to use the identical set of primers used in the synthesis of RNA, to produce RNA in a second cycle of TAS. As stated in the section describing the production of RNA, the primer pair should not overlap. In carrying out the method of the invention in which the production of RNA is desired, it is possible that the subsegment of target segment with sequence complementary to that of (5'-primer subsegment)$_4$ be in the 5'-direction from and not overlap the subsegment of target segment with sequence complementary to that of (3'-primer subsegment)$_1$. Similarly it is preferred that the subsegment of target segment with sequence the same as that of (3'-primer subsegment)$_3$ be in the 3'-direction from and not overlap the subsegment of target segment with sequence the same as that of (5'-primer subsegment)$_2$. This strategy may be preferred because it reduces the competition of the different primers for the same sites and, thereby, may markedly enhances the efficiency of amplification according to the invention. Where there is some overlap between sets of nested primers, it is preferable to remove any unused first set of primers, before employing the second set.

The focus of the present invention is on bacteriophage DNA-dependent RNA polymerase because of their high specificity for certain promoters. Other polymerase which have similarly high specificity for particular promoters could be employed in accordance with the invention in place of the bacteriophage polymerase and the invention is intended to cover such other polymerase as well.

The preferred of the bacteriophage DNA-dependent RNA polymerase are those from T7, T3 and SP6. Preferred promoters for use with these polymerase are described in the examples and claims, but numerous other promoters for these polymerase are known in the art and can be employed as well.

Further, polymerase from bacteriophages other than the preferred three, and promoters recognized by such other polymerase, can be employed in accordance with the invention.

It is preferred to employ for DNA polymerase in the methods of the invention reverse transcriptases and DNA polymerase which lack 5' to 3' exonuclease activity. It is most preferred that a single DNA polymerase be used for all steps in the methods. Most preferred of the DNA polymerase are the AMV reverse transcriptase and recombinant MMLV reverse transcriptase. Other polymerase are acceptable however, such as the heat-stable DNA polymerase from Thermus aquaticus (see Chien et al. J. Bacteriol. 127, 1550 (1976)), Sequenase™ brand recombinant T7 DNA polymerase from the U. S. Biochemicals Corp., Cleveland, Ohio, U.S.A., and the well known Klenow Fragment of E. coli DNA polymerase I, and calf thymus DNA polymerase alpha.

The "variable subsegments" that are optionally included in the various primers serve three functions. In fact, they might be more aptly termed "multipurpose subsegments." First, for the first and third primers, which include promoters, the promoter subsegments preferably include transcription initiation sequences that are preferred by the RNA polymerase corresponding to the promoter. Second, for all of the primers, a variable subsegment can optionally contain a particular segment whereby RNA product from the amplification can be detected in a nucleic acid probe hybridization assay. Indeed, amplification (and assay) can occur for several different target segments simultaneously by using sets of primers that differ in their recognition segments (e.g., (3'-primer subsegment)$_1$) but include a common variable subsegment. The variable subsegment can also contain a polylinker sequence that conveniently contains a plurality of restriction sites for ease of cloning subsequently. Finally, the variable subsegments can be used to incorporate into the RNA product from amplification sequences necessary to allow the RNA product to be (autocatalytically) replicated by a bacteriophage RNA-specific RNA polymerase, such as the Qβ replicase, see Miele et al., supra, and BMV sequences from the RNA-3 chromosome of the plant virus that promotes the synthesis of coat mRNA. See Ahlquist et al., J. Mol. Biol. 172, 369 (1984); Ahlquist et al., Plant Mol. Biol. 3, 37 (1984); Ahlquist et al., J. Mol. Biol. 153, 23 (1981); Miller, et al., Nature 313, 68 (1985); Miller et al., Virology 125, 236 (1983); and Ou et al., PNAS 79, 5235 (1982).

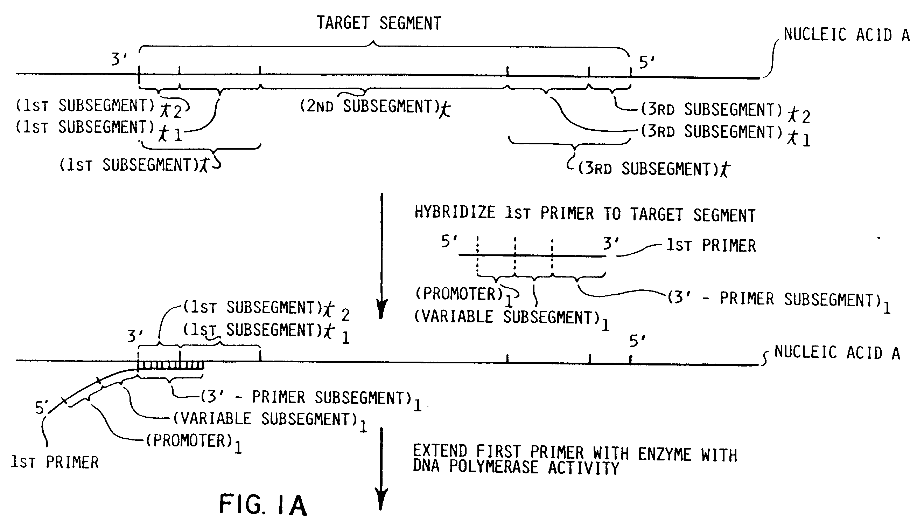

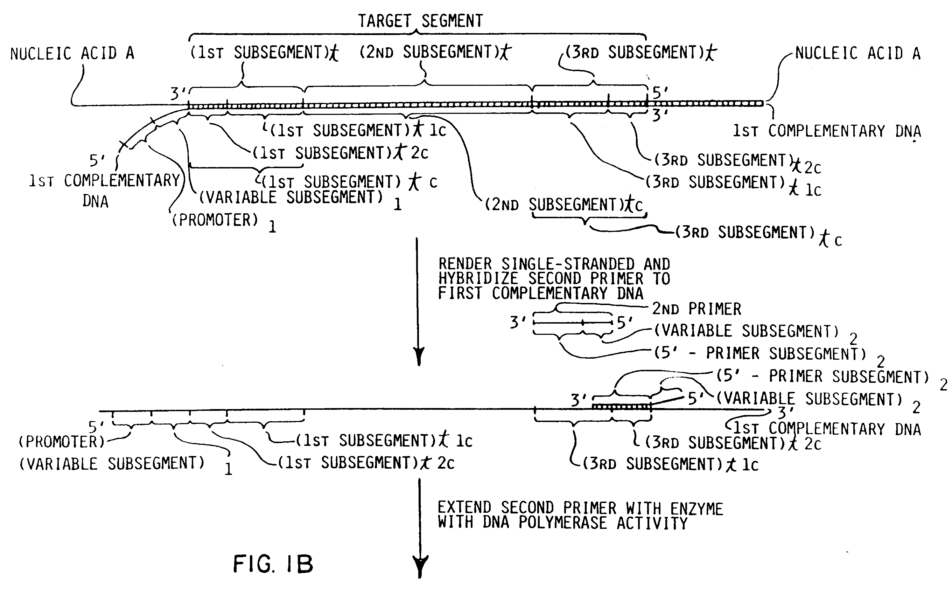

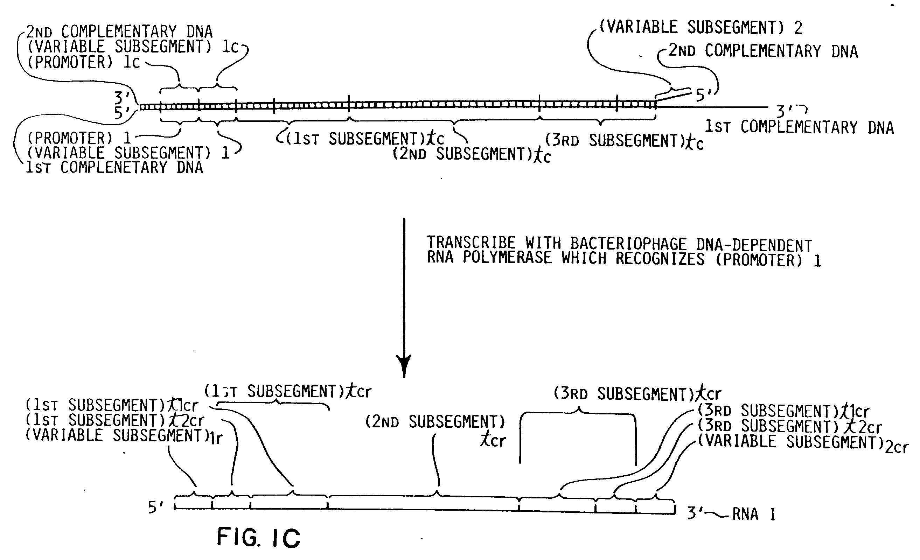

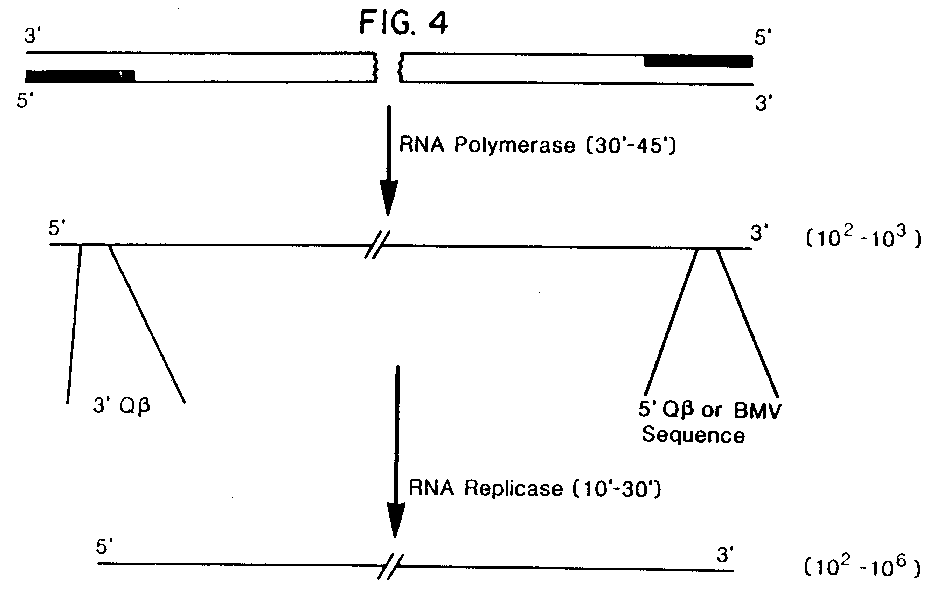

In the case of the Qβ replicase, which is known in the art, this is preferably carried out by including in (variable subsegment)$_2$ the sequence (SEQ ID No: 1) 5'-CGCGCTCTCCCAGGTGACGCCTCGAGAAGAGGCGCGACCTTCGTGC-3'and in (variable subsegment)$_1$ the sequence (SEQ ID No: 2) 5'-TGGGGAACCCCCCTTCGGGGGTCACCTCGCGCAGC-3', and then (autocatalytically) replicating the first RNA product (i.e., RNA. I in Figure 1; see also Figure 4), or including in (variable subsegment)$_4$ the sequence (SEQ ID No: 1) 5'-CGCGCTCTCCCAGGTGACGCCTCGAGAAGAGGCGCGACCTTCGTGC-3' and in (variable subsegment)$_3$ the sequence (SEQ ID No: 2) 5'-TGGGGAACCCCCCTTCGGGGGTCACCTCGCGCAGC-3', and then (autocatalytically) replicating the second RNA product (i.e., RNA II in Figure 1; see also Figure 4).

In the case of BMV replicase use, also known, this is preferably carried out by including the following sequence for BMV in variable sequence 2 (bases 1-25) (in the specific use of the HIV example infra) (SEQ ID No: 3):

```
     BMV promoter                    TCS = 87-29 (HIV-specific)


                .
                10        20        30        40
                 |         |         |         |
    5'-AAGATCTATGTCCTAATTCAGCGTA|ACAGCATATGTATGTTCAGGGA


    50
     |
  AAGCTA-3' (54 bases),
```

as a core sequence, additionally AU rich sequence 5'-thereof may be used to enhance the replicase activity from BMV. This oligo is to be used as the second primer. The first primer used with this second primer is a T7-based, HIV-specific primer (SEQ ID No: 4)

```
     T7 promoter                       TCS = 87-34


                        +1S
        10           |20          30        40         50
         |           ||           ||         |          |
   TAATACGACTCACTATA|GGGA|CACCTAGGGCTAACTATGTGTCCTAATAAGG

   (52 bases).
```

Additional, non-limiting details to aid in understanding the invention are provided in the examples that follow:

4. <u>Examples</u>

EXAMPLE I

10ml of blood from a patient suspected of being infected with a human immunodeficiency virus-type 1 (HIV-1) is fractionated with a Sepracell™ apparatus (Sepratech Corp., Oklahoma City, Oklahoma, U.S.A.) or, alternatively, with a Ficoll gradient, to isolate lymphocytes. The lymphocytes are then lysed and nucleic acid from them is isolated with an extractor apparatus (Molecular Biosystems, Inc., San Diego, California, U.S.A.) or, alternatively, the lymphocytes are lysed by standard sodium dodecyl sulfate (SDS)-enzyme treatment and the nucleic acid isolated with reverse phase chromatography over DEAE cellulose. This is done as follows:

Spin down cells: 5k rpm for 4' from 1 ml Tris-buffered saline (TBS), pH 7.5.
Draw off supernatant.
Resuspend pellet in:

| | |
|---|---|
| 500 µl 0.3 M NaCl/20 mM Tris, pH 7.5<br><br>100 µl 2% SDS<br><br>200 µl 5 mg/ml proteinase K<br><br>200 µl 0.25 M EDTA | Mix well, then add to pellet. |

Vortex vigorously and incubate at 50°C for 45', vortexing for 10-15 seconds every 10'.

Load onto drained extractor column and allow to enter.

Wash column 1x 4 ml 0.3 M NaCl/20 mM Tris, pH 7.5.

Elute DNA/RNA with 4 ml 0.5 M NaCl/20 mM Tris pH 7.5.

To eluate add:

    5 µl glycogen
    400 µl 3 M NaOAc
    9.0 ml ice-cold EtOH
    mix well

Precipitate at -20°C overnight. A dry ice/ethanol bath for one hour may be used.

Spin down DNA/RNA for 15' at 10,000 rpm in a swinging bucket rotor; pour off EtOH and drain dry on a kimwipe ˜2'.

Lyophilize for 10'.

Resuspend pellet in 170 µl TE.

Incubate at 37°C for 5'.

Pour a 1 ml spin column as follows:

    Plug a 1 ml syringe with a small amount of glass wool (autoclaved).
    Fill syringe with TE (tris-EDTA) treated with diethyl pyrocarbonate (DEPC).
    Quickly add sephadex G-50 fine (in TE; autoclaved).
    Continue to add until the solid matrix mounds over the top of the syringe.
    Spin 30 seconds at 1000 rpm in an IEC table-top centrifuge.
    Wash with 100 µl TE, spin for 1' at medium speed (ca. 1000 rpm). Discard wash.

Load the extract onto the column. Spin again for 1'.

Rinse with 150 µl TE, spin at ˜1000 rpm for 1'. Pool rinse and the first fraction. Samples may be split for multiple reactions at this step.

Add 1/10 volume 8 M LiCl. Mix with 2.5 x vol. 100% EtOH. Vortex well. ppt. dry ice/EtOH for 30'.

Spin down for 15' top speed in a microfuge at 4°C.

Dry pellet.

After the isolation, total nucleic acid from the sample is dissolved in 100µl of TE buffer (10mM Tris.Cl, 1mM EDTA, pH8). 10µl of the 100µl of total nucleic acid is dissolved to a final volume of 100µl containing:

    40mM Tris.Cl, pH8
    25mM NaCl
    10mM $MgCl_2$
    10mM dithiolthreitol (DTT)
    2mM spermidine
    100µg/ml bovine serum albumin
    400mM each of dATP, dCTP, dGTP and TTP
    30nM of the 101 base single-stranded DNA of sequence (SEQ ID No: 5) 5'-GAACGCGGCT ACAATTAATA CATAACCTTA TGTATCATAC ACATACGATT TAGGTGACAC TATA GAATAC TTTCGTAACA CTAGGCAAAG GTGGCTTTAT C -3' primer A, is added. 30nM of the 29 base DNA of sequence (SEQ ID No: 6) 5'-ACACCATATG TATGTTTCAG GGAAAGCTA-3', primer B, is added. Primer B, which is the second primer, has the sequence of bases 5151-5179 in the SOR gene of the HIV-1. An alternative segment for the second primer, corresponding to bases 5357-5387 of the SOR gene of the HIV-1, has the sequence (SEQ ID No: 7) 5'-GCACACAAGT AGACCCTGAA CTAGCAGACC A-3', and, if used, is also present at 30 nM. Primer A is the first primer: its 64 5'-nucleotides form the plus-strand of a promoter for the SP6 DNA-dependent RNA polymerase. Its segment of sequence 5'-GAATAC-3' is (alternative subsegment)$_1$, which includes the transcription start site (the 5'-G) and other bases apparently favored by the SP6 RNA polymerase at the 5'-end of sequences transcribed by it. Finally, the 31 3'-terminal nucleotides form (primer subsegment)$_1$ and have a sequence complementary to that of bases 5577-5547 in the SOR gene of an HIV-1 isolate (See Ratner et al., Nature 313, 277 (1985) for complete sequence.) (The HIV isolate is referred to in these Examples as "the HIV-1")).

Note that all oligonucleotides employed in these examples are made by solid-phase synthesis on an Applied Biosystems, Inc. (Foster City, California, U.S.A.) automated synthesizer (Model No. 380A) and are purified chromatographically essentially to homogeneity by HPLC using a C8 reverse phase column. Alternatively, other commercially available synthesizers and standard purification procedures could be used to prepare the oligonucleotides.

The 100µl of solution is heated at 65°C for 2 minutes and is then cooled to 42°C over the course of 1 minute. This heating and then holding at 42°C or above, in combination with the composition of the solution, provides conditions of stringency sufficient to provide hybridization of (3'-primer subsequent)$_1$, with sufficient stability to prime DNA synthesis, of high specificity to the sequence complementary to that of (3'-primer subsequent)$_1$.

Then 10 units of avian myoblastosis virus (AMV) reverse transcriptase or 500 units of recombinant Moloney murine leukemia virus (MMLV) reverse transcriptase, as purchased from Life Sciences, Inc., St. Petersburg, Florida, U.S.A., are added to the solution and the solution is incubated for 10 minutes at 42°C. (One unit incorporates 1nmole of TTP into acid-precipitable form in 10 minutes at 37°C using poly(A).oligo(T)$_{12-18}$ as template-primer as defined by Houts et al., J. Virol. 29, 517 (1979)). After the 10 minute incubation, the solution is placed in a boiling water bath for 1 minute. This heating causes strand-separation of the duplex formed by the reverse transcriptase.

Then the solution is cooled to 42°C over 1 minute. During this cooling, the second primer hybridizes to the 3'-terminal segment of the complement of target segment formed in the reaction catalyzed by the reverse transcriptase. Again, the hybridization conditions are sufficiently stringent for sufficiently specific hybridization between the second primer and sequence complementary to that of second primer.

After the cooling, 10 additional units of AMV reverse transcriptase or 500 additional units of cloned MMLV reverse transcriptase are added and further incubation for 10 minutes at 42°C is carried out.

Then RNasin$^R$ brand ribonuclease inhibitor from Promega Biotec, Madison, Wisconsin, U.S.A. is added (optionally) to a concentration of 1 unit per ml. (1 unit is the amount of inhibitor required to inhibit by 50% the activity of 5ng of ribonuclease A. Roth, Meth. Cancer Res. 3, 151 (1976)). Further, each of the ribonucleoside 5'-triphosphates, ATP, GTP, CTP and UTP, is added to 400mM. Finally between 10 and 20 units of SP6 DNA-dependent RNA polymerase, purchased from Promega Biotec, are added and the resulting solution is incubated at 42°C for 30 to 60 minutes. (1 unit is the amount of the RNA polymerase required to catalyze the incorporation of 1 nmole of ribonucleoside triphosphate into acid insoluble product in 60 minutes at 37°C under standard reaction conditions (40mM Tris.Cl, pH 7.9, 6mM MgCl$_2$, 10mM DTT, 2mM spermidine, 0.5 mM of each of ATP, GTP, CTP and UTP, 0.5Ci of $^3$H-CTP, 1g of SP6 DNA and the enzyme in a total volume of 50µl.))

Then each of the following oligonucleotides is added to bring its concentration to 30nM:

third primer (SEQ ID No: 8):
5'-GAACGCGGCT ACAATTAATA CATAACCTTA TGTATCATAC ACATACGATT TAGGTGACAC TATA GAATAC ACTAATTCAT CTGTATTACT TTGACTGTTT TTC-3'
fourth primer (SEQ ID No: 9):
5'-TTTTTTGGTG TTATTAATGC TGCTAGTGCC-3'

In the third primer, as in the first, the 5'-terminal 64 bases are the promoter segment and the next 6 bases are the variable segment. The variable segment is the first six bases transcribed from the promoter by the SP6 RNA polymerase, and these bases are selected to enhance the level of such transcription. Finally, the (3'-primer subsegment)$_3$ portion of the third primer is the 3'-terminal 33 bases, in the same sequence as bases 5388-5420 in the short open reading frame (SOR) gene of the HIV-1. The fourth primer has the sequence complementary to that of bases 5546-5517 of the SOR gene of the HIV-1.

After addition of the third and fourth primers, the solution is incubated at 42°C for 1 minute. During this period, hybridization occurs between (3'-primer subsegment)$_3$ of third primer and the first RNA formed in the reaction catalyzed by the SP6 RNA polymerase. Because of the stringency of the hyhridization conditions, the (3'-primer subsegment)$_3$ hybridizes, with stability sufficient for priming of DNA synthesis, with high specificity to the segment of complementary sequence in the first RNA.

After the incubation, 10 units of AMV reverse transcriptase or 500 units of cloned MMLV reverse transcriptase are added and the solution incubated for 10 minutes at 42°C to form the third complementary DNA.

The solution is then suspended in a boiling water bath for 1 minute and cooled to 42°C over 1 minute to, first, render single-stranded the duplex between third complementary DNA and first RNA and, second, allow hybridization between fourth primer and third complementary DNA. As with the other hybridizations, the conditions are sufficiently stringent that hybridization of fourth primer, with stability sufficient to prime DNA synthesis, occurs with high specificity to the segment of third complementary DNA of sequence complementary to that of fourth primer.

Then, again, 10 units of AMV reverse transcriptase or 500 units of cloned MMLV reverse transcriptase are added and the solution is incubated for 10 minutes at 42°C.

Then RNasin$^R$ brand ribonuclease inhibitor is added (optionally) to 1 unit per ml, followed by 10-20 units of SP6 RNA polymerase, and the solution is incubated for 30 minutes to 1 hour at 42°C.

The resulting second RNA can then be detected by a nucleic acid probe hybridization technique.

EXAMPLE II

The procedure of Example I is followed, with a modification noted below, with three samples: (A) one of 10ml of human blood known to be free of HIV, (B) one of 10ml of culture known to have about $10^3$ HIV-1 infected cells per ml, and (C) one of 10ml of blood from a person suspected of being infected with an HIV-1. The modification of the procedure is that an alpha-$^{32}$P-labeled ribonucleoside triphosphate is included as a substrate in the reaction catalyzed by the SP6 RNA polymerase to make the second RNA. The second RNA is, consequently, $^{32}$P-labeled.

Sephacryl-S500™ macroporous beads are derivatized with a carboxyl-group-terminated linker (of formula-$C(=NH)NH(CH_2)_5 CO_2$-) and then with 5'-(6-aminohexyl phosphoramidate)-derivatized oligonucleotide of sequence (SEQ ID No: 10) 5'-TGGTCTGCTA GTTCAGGGTC TACTTGTGTG C-3' which is the sequence complementary to that of bases 5357-5387 in the SOR gene of the HIV-1. (The sequence of bases 4901-4932 of the SOR gene occurs in any second RNA produced during amplification of nucleic acid from a sample.) Preparation of the beads was according to procedures described in commonly assigned United States Patent Application Serial No. 895,756, filed August 11, 1986, and incorporated herein by reference. Briefly, the support materials are porous silicate glass or macroporous cross-linked dextran, derivatized with an amino-terminated linker, with substantially all of the amino groups that are not covalently joined through a phosphoramidate group to the terminal nucleoside of the capture probe being blocked from interacting non-specifically with nucleic acid having an aliphatic acyl group; macroporous cross-linked dextran activated with cyanogen bromide, which reacts with amines to link to aminoalkylphosphoramidate derivatized oligonucleotides; and porous silicate glass, macroporous cross-linked dextran or divinylbenzene-crosslinked polystyrene derivatized with a carboxyl-terminated or succinimide-ester-terminated linker, with a fraction of the carboxyl groups joined in an amide linkage to one amino group of a diaminoalkane, the other amino of which is part of a phosphoramidate which, in turn, is bonded directly to the terminal nucleoside of the capture probe. Preferred support materials are long chain alkylamine-derivatized and carboxyl-derivatized controlled pore glass as sold by Pierce Chemical Co., Rockford, Illinois, USA, under product numbers 24875 and 23735, respectively, in the form of beads with nominal pore diameter of 500 Angstroms and particle diameters between about 125 and 177 microns, and Sephacryl S-500, sold by Pharmacia Inc., Piscataway, New Jersey, USA under Code No. 17-0475-01 in the form of beads with a wet diameter of about 40 to about 105 microns and an exclusion volume for dextrans of molecular weight above about $2\times10^7$ daltons, said Sephacryl to be derivatized with cyanogen bromide or with an amino-terminated or carboxyl-terminated linker. In one aspect the solid support is one of:

(A) porous silicate glass derivatized at silicons with

(1) groups of formula

$$-(CH_2)_n(NH)(CO)(CH_2)_cCH_3$$

and

$$-(CH_2)_n(NH)(PO_2)0-(Oligo),$$

with substantially no silicon derivatized with a group terminated with an amino group, wherein c is 0 to 5, n is 2 to 8, -0-(Oligo) is the oligonucleotide probe, and the oxygen atom bonded to (Oligo) is the oligonucleotide probe, and the oxygen atom bonded to (Oligo) is the 5'-oxygen of the 5'-nucleoside or the 3'-oxygen of the 3'-nucleoside of the probe; or

(2) groups of formula

$$-(CH_2)_n(NH)(CO)(CH_2)_mCO_2H$$

and

$$-CH_2)_n(NH)(CO)(CH_2)_m(CO)(NH)(CH_2)_pNH(PO_2)O-(Oligo),$$

wherein m, n, and p are the same or different and are each 2 to 8; or

(B) a cross-linked dextran macroporous material derivatized at hydroxyl oxygens, which are on carbons of the sugar moieties which have at least one neighboring carbon with an underivatized hydroxyl, with

(1) groups of formula

$$-C(=NH)NH(CH_2)_q(NH)(CO)(CH_2)_cCH_3$$

and

$$-C(=NH)NH(CH_2)_p(NH)(PO_2)O\text{-Oligo}),$$

with substantially no hydroxyl oxygen derivatized with a group terminated with an amino group, or
(2) groups of formula

$$-C(=NH)NH(CH_2)_qCO_2H$$

and

$$-C(=NH)NH(CH_2)_q(CO)(NH)(CH_2)_pNH(PO_2)O\text{-(Oligo)},$$

wherein c, q, and p are the same or different and q is 2 to 10; and

(C) divinylbenzene-crosslinked polystyrene derivatized at phenyl groups with groups of formula

$$-(CH_2)_sCO_2H$$

and

$$-CH_2)_s(CO)(NH)(CH_2)_pNH(PO_2)O\text{-(Oligo)},$$

wherein s and p are the same or different and s is 2 to 10. See Ghosh et al., Nucleic Acids Research 15, 5353 (1987).

Each of three batches of 50mg of Sephacryl beads, derivatized as described above, is soaked for 15 minutes at 37°C C in 250μl of a prehybridization solution containing 0.1% SDS, 10% dextran sulfate, 1mg/ml salmon sperm DNA, and 5x SSPE (0.75M NaCl, 50mM NaH$_2$PO$_4$, pH 7.4, and 5mM EDTA). After the soaking, the bead material is pelleted by centrifugation and prehybridization solution is removed by aspiration.

The nucleic acid from the amplification procedure on each of the three samples is isolated by ethanol precipitation and is then dissolved to 250μl in solution that is the same as pre-hybridization solution but lacks the salmon sperm DNA. Each of the resulting nucleic acid solutions is then combined with one batch of prehybridized oligonucleotide-derivatized Sephacryl beads and the resulting mixture is incubated with gentle agitation at 42°C C for 90 minutes.

The Sephacryl beads are then pelleted by centrifugation, hybridization solution is removed by aspiration, and the beads are then washed three times, 10 minutes each at 37°C C, in 1ml of a solution of 2 x SSC (0.30M NaCl, 0.03M Na.Citrate, pH 7.0). After each wash, the beads are pelleted by centrifugation and solution is removed by aspiration.

Immediately after the third wash, the beads are subjected to Cerenkov counting.

The beads with amplified nucleic acid from sample A produce a low level of counts, barely above background counts from scintillation fluid alone. The beads with amplified nucleic acid from sample B produce counts at a much higher level than that observed with beads associated with sample A. The beads with amplified nucleic acid from sample C, if they produce a level of counts significantly above the level produced with beads associated with sample A, indicate that the person, whose blood was taken for sample C, is infected with HIV-1.

EXAMPLE III

The procedures of Examples I and II are carried out with T7 RNA polymerase (Promega Biotec) in place of SP6 RNA polymerase, with each of the subsegment 5'-(promoter)$_1$-(variable subsegment)$_1$-3' of first primer and the subsegment 5'-(promoter)$_3$-(variable subsegment)$_3$-3' of third primer having the sequence (SEQ ID No: 11) 5'-TAATACGACT CACTATA GGGA-3', wherein the 17 5'-terminal bases are the promoter subsegment and the 4 3'-bases are the variable subsegment. The variable segment corresponds to the 4 5'-terminal bases of the transcript from the promoter. Ribonuclease inhibitor is not used in any step in this process, at least with the polymerase preparation from Promega Biotec.

EXAMPLE IV

The procedures of Examples I and II are carried out with T3 RNA polymerase (from Stratagene, San Diego, California) in place of the SP6 RNA polymerase and with the following segment used as the (promoter)$_1$-(variable sub-segment)$_1$ subsegment of first primer and the (promoter)$_3$-(variable subsegment)$_3$ subsegment of third primer (SEQ ID No: 12): 5'-TATTAACCCT CACTAAA GGGA-3', where the 17 5'-terminal bases are the promoter segment and the 4 3'-terminal bases the variable segment, including the 5'-terminal 4 bases in transcripts from the promoter.

EXAMPLE V

Employing the T7 RNA polymerase as in Example III, target segment in the genome of HIV from human blood samples is amplified using the following primers in place of the primers specified in Example I:

First Primer (SEQ ID No: 13): 5'-TAATACGACT CACTATA GGGA TCTAATTACT ACCTCTTCTT CTGCTAGACT-3', wherein the 30 3'-terminal bases are complementary in sequence to bases 7076-7047 of the ENV gene of HIV-1.

Second Primer (SEQ ID No: 14): 5'-ACAAGTTGTA ACACCTCAGT CATTACACAG-3' with the sequence of bases 6838-6866 in the ENV gene of HIV-1.

Third Primer: Same 21 5'-terminal bases as first primer of this Example adjoined to the following 27 3'-terminal bases (SEQ ID No: 15): 5'-AAAGGTATCC TTTGAGCCAA TTCCCATA-3', which have the sequence of bases 6875-6902 in the ENV gene of the HIV-1.

Fourth Primer (SEQ ID No: 16): 5'-AGTTGATACTACTGGCCTAATT-3', with the sequence complementary to that of bases 7033-7007 in the ENV gene of HIV-1.

EXAMPLE VI

Employing the T7 RNA polymerase as in Example III, target segment in the genome of HIV from human blood samples is amplified using the following primers in place of the primers specified in Example I:

First Primer: Same 21 5'-terminal nucleotides as the first and third primers of Example V adjoined to the following 31 3'-terminal nucleotides (SEQ ID No: 17): 5'-CACCTAGGGC TAACTATGTG TCCTAATAAG G-3', which have the sequence complementary to that of bases 5471-5441 of the SOR gene of HIV-1.

Second Primer (SEQ ID No: 18): 5'-ACACCATATG TATGTTTCAG GGAAAGCTA-3', which has the same sequence as bases 5151-5179 of the SOR gene of HIV-1.

Third Primer: Same 21 5'-terminal nucleotides as the first and third primers of Example V adjoined to the following 31 3'-terminal nucleotides (SEQ ID No: 19): 5'-AAGAATAAGTTCAGAAGTACACATCCCACT-3', which have the same sequence as bases 5220-5249 of the SOR gene of the HIV.

Fourth Primer (SEQ ID No: 20): 5'-TGGTCTGCTAGTTCAGGGTCTACTTGTGTC-3', which has the sequence complementary to that of bases 5387-5357 in the SOR gene of HIV-1.

EXAMPLE VII

The procedure of Example I is followed for the isolation of nucleic acid from: 1) one sample containing $10^3$ HIV-infected CEM cells mixed with $10^6$ uninfected CEM cells (Cancer Center Research Foundation; CCRF-CEM; ATCC No. CCL 119); and 2) one sample containing $10^6$ uninfected CEM cells. These samples are resuspended in 100 μl 10 mM Tris-HCl, pH 7.4, 1 mM EDTA (TE) after the ethanol precipitation step to concentrate the sample obtained from the Extractor™ column (MBI). The resuspended samples are fractionated on a Sephadex G-50 fine spin column (Maniatis supra) eluting with TE. The eluted samples are concentrated by ethanol precipitation (in 0.8 M LiCl, 3 volumes ethanol, in dry ice/ethanol bath for 15 minutes). The precipitated sample is pelleted by centrifugation. The pellet is drained, dried, and then resuspended in 100 μl containing 40 mM Tris-HCl, pH 8.1, 8 mM $MgCl_2$, 25 mM NaCl, 2 mM spermidine, 5 mM dithiothreitol, 100 μM each dATP, dGTP, dCTP, and dTTP, 1 mM each rATP, rCTP, rGTP, and rUTP, 100 μg/ml BSA (nuclease-free) and 250 nM each of DNA oligonucleotide primer A

(SEQ ID No: 21)

(5'-AATTTAATACGACTCACTATAGGGACACCTAGGGCTAACTATGTCCTAA-

TAAGG-3)

and primer B (SEQ ID No: 22) (5'-ACACCATATGTATGTTTCAGGGGAAAGCTA-3').

As controls, duplicate samples of purified HIV RNA at a concentration of 0.01 fm were resuspended in 100 μl of the buffer described above. Finally, a 10-fm β-globin sequence contained in plasmid Hβ19A [Wallace et al., Nucl. Acids Res. 9, 3647 (1981), which had been linearized by HindIII, was resuspended in 100 μl of the buffer described above, except without the oligonucleotide primers. Oligonucleotide primer (SEQ ID No: 23) (5'-ACATTGCTTCTGACACAACTGTGTTCA-') and primer C (SEQ ID No: 24) (5'-TAATACGACTCACTATAGGGACAAAGGACTCAAA-3'), which are specific for β-globin sequence, were added to the β-globin reaction at 250 nM each.

Except for the β-globin sample, the reactions are heated to 65°C for 1'. The β-globin sample is boiled for 1'. All samples are cooled to 42°C for 1 minute; then, 10 units of AMV reverse transcriptase are added. The reactions are incubated for 15 minutes at 42°C, boiled for 1 minute, and then cooled at 42°C for 1 minute. Ten additional units of AMV reverse transcriptase are added, incubating at 42°C for 15 minutes. One hundred units of T7 RNA polymerase are added to the reactions, and the reactions are incubated at 37°C for 30 minutes.

The samples are boiled for 1 minute and cooled at 42°C for 1 minute, followed by the addition of 10 units AMV reverse transcriptase. The reactions are incubated at 42°C for 15 minutes, boiled for 1 minute and cooled to 42°C for 1 minute. An additional 10 units of reverse transcriptase are added, followed by incubation at 42°C for 15 minutes. One hundred units of T7 RNA polymerase are added, with a 30-minute incubation at 37°C. This cycle is repeated two additional times.

The amplified target is then detected using OligoBeads™ as described below.

Sephacryl beads containing HIV-1-specific oligonucleotides were prepared as described and stored in TE at 4°C at a concentration such that 250μl of the suspension contains 50mg of Sephacryl beads. Oligonucleotides were synthesized and HPLC purified as described.

The oligonucleotides employed for both attachment to the beads and detection are homologous to the SOR region of the HIV-1 genome. The oligonucleotides used in these studies were 86-31 (detection oligo nucleotide) (SEQ ID No: 25): (5'-GCACACAAGTAGACCCTGAACTAGCAGACCA-3') and 87-83 (capture oligo nucleotide on the bead (SEQ ID No: 26): 5'-ATGCTAGATTGGTAATAACAACATATT-3'), which are homologous to the nonsense strand of the SOR region, and separated by approximately 100 nucleotides. For detection, the oligonucleotides were end labeled with $^{32}$-P according to a standard protocol. The unincorporated label was removed by gel filtration on a Sephadex G-50 fine column, and the oligonucleotide was stored at -20°C.

In a typical bead based sandwich hybridization system (BBSHS) experiment, the target and $^{32}$P-labeled detection oligonucleotide are denatured in 10μl of TE containing 0.2% SDS at 65°C for 5 minutes in an Eppendorf tube. To this, 10μl of 2X Solution Hybridization Mix (10X SSPE/10% Dextran Sulfate) are added. The solution is mixed, centrifuged 2 seconds, and incubated at 42°C for 1 hour.

During this time the Sephacryl beads are prehybridized. The stock suspension of beads is mixed well, and 250μl aliquots (50 mg of beads) are transferred to Eppendorf tubes with mixing between each removal to ensure a uniform suspension. The aliquots are centrifuged for 10 seconds, and the TE is removed with a drawn-out Pasteur pipet. After 250μl of Hybridization Solution (5X SSPE [0.9M NaCl, 50 mM NaH$_2$PO$_4$, pH 7.4, 1mM EDTA] 10% Dextran Sulfate/0.1% SDS) is added, the beads are suspended by gentle shaking and incubated 37°C for 30-60 minutes with occasional mixing. Immediately prior to the capture step, the beads are centrifuged for 10 seconds, the prehybridization solution is removed, 80μl of Hybridization Solution at 37°C are added, and the beads are returned to 37°C.

The solution hybridization is centrifuged for 2 seconds and transferred to the beads and Hybridization Solution. The beads are suspended and incubated at 37°C for 1 hour with frequent mixing to maintain the suspension.

Following the capture, the beads are centrifuged 10 seconds and the Hybridization Solution, containing uncaptured target and detection oligonucleotide, is transferred to a scintillation counter vial. The beads are then washed 5 times with 2X SSC at 37°C. The first 3 washes are rapid; 1 ml of wash is added, the beads are mixed well and centrifuged 10 seconds, and the wash is transferred to a counting vial. For the final 2 washes, 1 ml of wash is added and the beads are mixed and incubated at 37°C for 5 minutes before being centrifuged. Each wash is counted separately to monitor the procedure.

Cerenkov counts of the Hybridization Solution, 5 washes, and beads are measured for 5-10 minutes. Counter background is subtracted from all samples. The fm target detected is calculated as follows:

(CPM on Beads/Total CPM) X fm Oligonucleotide

Where total CPM is the sum of the CPM for the Hybridization Solution, 5 washes, and beads.

| DETECTION OF AMPLIFIED TARGETS BY BBSHS | | |
|---|---|---|
| TARGET | µl USED | (FM) DETECTED $10^{-15}$ MOLES |
| $10^3$ HIV infected cells; | 0.33 | 0.06 |
| $10^6$ CEM uninfected cells | 0.66 | 0.13 |
| $10^6$ uninfected CEM cell | 0.07 | 0.01 |
| | 0.14 | 0.015 |
| | 0.7 | 0.01 |
| | 10 | 0.008 |
| 0.01 fm HIV RNA | 0.007 | 0.14 |
| | 0.014 | 0.29 |
| 10 fm β-globin DNA | 10 | 0.014 |

EXAMPLE VIII

The procedure of Example I is followed for isolation of nucleic acids from two samples: a) $10^3$ HIV-infected CEM cells with $10^6$ uninfected CEM cells; and b) $10^6$ uninfected cultured CEM cells to which 0.01 fmoles of purified HIV are added after extraction. The procedure is then modified by further purification through a Sephadex G-50 (fine) spin column (Maniatis supra) eluting with TE after the Extractor column step. This is followed by an ethanol precipitation of the nucleic acid (in 0.8 M LiCl and 2.5 volume of EtOH). The nucleic acid is then resuspended in 100 µl containing:

    40 mM Tris-HCl, pH 8.1
    8 mM $MgCl_2$
    25 mM NaCl
    2 mM spermidine
    5 mM DTT (dithiothreitol)
    100 µM dATP, dTTP, dCTP, dGTP (each)
    1 mM rATP, rCTP, rGTP, rUTP
    100 µg/ml BSA nuclease-free
    250 nM 29 bp DNA oligonucleotide with the sequence (SEQ ID No.6) 5'ACACCATATGTATGTTTCAGGGAAAGCTA-3' (Primer B)
    250 nM 56 bp DNA oligonucleotide with the sequence

```
 (SEQ ID No: 21) 5'-
AATTTAATACGACTCACTATAGGGACACCTAGGGCTAACTATGTGTCCTAA
         TAAGG-3') (Primer A)
```

The 100 µl of solution are heated to 65°C for 1 minute and cooled to 42°C over the course of 1 minute. This heating and then holding at 42°C or above, in combination with the composition of the solution, provides conditions of stringency sufficient for hybridization of (3'-primer subsegment)$_1$, (see Figure 1) with sufficient stability to the sequence complementary to that of (3'-primer subsegment)$_1$, in the target HIV RNA with high specificity.

Then, 10 units of avian myoblastosis virus (AMV) reverse transcriptase (Life Sciences, Inc.) are added to the solution, and the solution is incubated for 10 minutes at 42°C. [One unit incorporates 1 nmole of TTP into acid-precipitable form in 10 minutes at 37°C using poly(A)-oligo (T)12-18 as template-primer, as defined by Houts et al. J. Virol. 29, 517, (1979)]. After a 10 minute incubation, the solution is placed in a boiling water bath for one minute. This heating causes strand separation of the duplex formed by reverse transcriptase and inactivation of the reverse transcriptase.

Then, the solution is cooled to 42°C over one minute. During this cooling, the second primer, primer B, hybridizes to the 3'-terminal end (third subsegment)$_{t2c}$, see Figure 1, of the target complement of DNA strand formed in the reaction catalyzed by the reverse transcriptase in the previous step. Again, the hybridization conditions are sufficiently stringent for specific hybridization between the second primer and the sequence complementary to that of the second primer.

After cooling, 10 additional units of AMV reverse transcriptase are added and further incubation is carried out for 10 minutes at 42°C.

One hundred units of T7 polymerase are added to the reaction. The reaction is then incubated at 37°C for 30 minutes. An RNA transcript is synthesized which is complementary to the original target HIV RNA starting from the T7 promoter present at the 5' end of the duplex DNA template newly synthesized by the reverse transcriptase. The transcript has the sequence 5'-GGGA, then continues with sequence which is complementary to the second subsegment of the target sequence (_i.e._, second subsegment$_{tcr}$, see Figure 1). Since the reverse transcriptase has not been inactivated prior to this step, and since primer B is present, as well as the deoxynucleotide triphosphates, a secondary synthesis occurs at this stage. Primer B hybridizes to the 3' region of the newly synthesized RNA transcript (third subsegment)$_{t2cr}$, see Figure 1, which is complementary to B primer. The reverse transcriptase ( which had been added at the previous step) synthesizes a DNA strand using the newly synthesized RNA transcript (made by T7 polymerase) as a template, and using oligonucleotide B as the primer at the 5' end.

The reaction is then boiled for one minute to denature the RNA:DNA duplex. The reaction is then cooled to 42°C over one minute. During this cooling step, the target complementary segment of primer A hybridizes to the DNA strand synthesized during the previous step. At the same time, primer B hybridizes to its complementary sequence on the RNA transcript synthesized in the previous step.

After cooling, 10 additional units of AMV reverse transcriptase are added, and further incubation for 10 minutes at 42°C is carried out. Reverse transcriptase synthesizes DNA complementary to the HIV target using oligonucleotide A as a primer at the 5' end and DNA made in the previous step as a template. A second DNA strand is synthesized using oligonucleotide B as a primer and the RNA transcript made in the previous step as a template.

The reaction is boiled for one minute to denature the DNA duplex and the RNA:DNA duplex. The reaction is cooled to 42°C over one minute. Primer A hybridizes to the cDNA made using the RNA transcript as template in the previous step. (If the 3' end of the template strand is used as a reverse transcriptase primer also, a product identical to the end product of the next reaction is produced.) Primer B hybridizes to the DNA strand which is complementary to the target HIV RNA. This second synthesis produces a product which is a duplex DNA containing a T7 promoter sequence at its 5' end, as well as a 4 bp "variable" segment, 5'-GGGA-3'.

One hundred units of T7 polymerase are added to the reaction. The reaction is incubated for 30 minutes at 37°C. The T7 RNA polymerase uses the double-stranded duplex DNA containing a duplex T7 promoter as a template to transcribe an RNA which is complementary to the target second subsegment containing the additional sequence 5'-GGGA-3' at its 5' end.

This cycle (boil 1', 1' at 42°C, RT 10' at 42°C, RT 10'at 42°C, T7 polymerase 37°C 30') can be repeated as many times as needed to achieve the desired amplification of target sequence. The resulting product can then be detected by a nucleic acid probe hybridization technique.

EXAMPLE IX

Purified HIV RNA at a concentration of 1 fmole was resuspended in:

40 mM Tris-HCl, pH 8.1
8 mM MgCl$_2$
25 mM NaCl
2 mM spermidine
5 mM dithiothreitol
100 µM dATP, dTTP, dCTP, dGTP (each)
1 mM each rATP, rCTP, rGTP, rUTP
100 µg/ml BSA nuclease-free
250 nM 29 bp DNA oligonucleotide with the sequence (SEQ ID No: 6) 5'-ACACCATATGTATGTTTCAGGGAAAGCTA-3' (primer B)
250 nM 56 bp DNA oligonucleotide with the sequence

(SEQ ID No: 21) 5-'AATTTAATACGACTCACTATAGGGACACCTAGGGCTAACTA
TGTGTCCTAATAAGG-3' (primer A)

The 100 μl of solution are heated to 65°C for 1 minute and cooled to 42°C over the course of 1 minute. This heating and cooling step, in combination with the composition of the solution, provides conditions of stringency sufficient for the hybridization of primer A with sufficient stability to the sequence complementary to that of the primer A region in the target HIV RNA [(first subseqment)$_{t2}$, in Figure 1] with high specificity.

Then, 10 units of avian myoblastosis virus (AMV) reverse transcriptase (Life Science, Inc.) are added to the solution, and the solution is incubated for 10 minutes at 42°C. After a ten-minute incubation, the solution is placed in a boiling water bath for one minute. This heating causes strand separation of the duplex formed by reverse transcriptase and inactivation of the reverse transcriptase.

The solution is cooled to 42°C over one minute. During this cooling, the second primer B hybridizes to the 3' end of the newly synthesized target-complementary strand [or (third subsegment)$_{t2c}$ in Figure 1]. Again, the hybridization conditions are sufficiently stringent for specific hybridization between the second primer and the sequence complementary to that of the second primer.

After cooling, 10 additional units of AMV reverse transcriptase are added and further incubation is carried out for ten minutes at 42°C.

One hundred units of T7 RNA polymerase (New England Biolabs) are added to the reaction. The reaction is then incubated at 37°C for 30 minutes. An RNA transcript is synthesized which is complementary to the original target HIV RNA starting from the T7 promoter present at the 5' end of the duplex DNA template synthesized by the reverse transcriptase. The RNA transcript has the sequence 5'-GGGA, then continues with the sequence which is complementary to the second subsegment of the target sequence (i.e., second subsegment$_{tcr}$, Figure 1). Since the reverse transcriptase has not been inactivated prior to this step, and since primer B is present as well as the deoxynucleotide triphosphates, a secondary synthesis occurs at this stage. Primer B hybridizes to the 3' region of the newly synthesized RNA transcript [(third subsegment)$_{t2cr}$, see Figure 1] which is complementary to primer B. The reverse transcriptase (which had been added at the previous step) synthesizes a DNA strand using the newly synthesized RNA transcript (made by the T7 RNA polymerase) as a template, and oligonucleotide B as a primer at the 5' end.

The reaction is then boiled for one minute to denature the RNA:DNA duplex. The reaction is then cooled to 42°C over one minute. During this cooling step, the target-complementary segment of primer A hybridizes to the DNA strand synthesized in the previous step. At the same time, primer B hybridizes to its complementary sequence on the RNA transcript synthesized in the previous step.

After cooling, 10 units of AMV reverse transcriptase are added, and further incubation for 10 minutes at 42°C is carried out. Reverse transcriptase synthesizes DNA complementary to the HIV target using oligonucleotide A as a primer at the 5' end and DNA made in the previous step as a template. The 3' end of the template strand is used as a primer to produce a final duplex DNA with a double-stranded, T7 promoter site at its 5' end. A second DNA strand is synthesized using oligonucleotide B as a primer and the RNA transcript made in the previous step as a template.

One hundred units of T7 polymerase are added to the reaction. The reaction is incubated for 30 minutes at 37°C. The T7 RNA polymerase synthesizes an RNA transcript using the duplex DNA containing the polymerase binding site (T7 promoter) at its 5' end as a template. The RNA transcript is complementary to the target second subsegment, see Figure 1, containing the additional sequence 5'-GGGA-3' at its 5' end. Further cycles may be performed to increase the amplification. The resulting products can be detected by a nucleic acid hybridization protocol.

EXAMPLE X

Protocol for Last-Round Labeling of a TAS Amplified Product

A. Column to remove unincorporated cold nucleotides

1. The TAS reaction (100 μl) is taken after the cDNA synthesis in the final cycle of TAS. Add 400 μl of Reagent A (0.1 M Tris-HCl, pH 7.7, 10 mM triethylamine, 1 mM disodium EDTA) to the reaction.
2. Equilibrate a NENSORB$_{20}$™ (Dupont) prepac column by wetting the column material in 100% methanol (HPLC grade), then equilibrating with 2 ml Reagent A.

3. Load sample on column.

4. Wash the column 3 times with 1 ml Reagent A.

5. Wash the column 3 times with 1 ml water.

6. Elute the nucleic acids with 50% methanol, collecting 250-300 µl fractions up to 1 ml total volume. (The first two fractions will contain the majority of the nucleic acid.)

7. Dry the fractions in the speed-vac or lyophilizer.

B. Protocol to Label the Amplified Product

1. Resuspend the fractions from the NENSORB$_{20}$™ column (the first two fractions may be combined) in 40 mM Tris-HCl, pH 8.1, 8 mM MgCl$_2$, 25 mM NaCl, 2 mM spermidine-(HCl)$_3$, 5 mM dithiothreitol, 400 µM each rATP, rCTP, and rGTP, 12 µM rUTP, and 25 µCi a-32p-rUTP (800 Ci/mmol).

2. Add 50 units T7 RNA polymerase (New England Biolabs) for each 50 µl of sample. Incubate at 37°C for 30 minutes.

3. The unincorporated label may be removed by a G-50 spin column (Maniatis supra).

4. The labeled sample may be run on a sequencing polyacrylamide gel to determine the size of the amplified product. The labeled product may also be detected using the Oligo-Beads™.

EXAMPLE XI

Use of an Internal Standard During TAS Amplification

Samples were prepared having: 1) 0.1 fm HIV RNA and 0.1 fm β-globin DNA sequences (Hβ19A cleaved with PstI); 2) 0.01 fm HIV RNA and 0.1 fm β-globin DNA (Hβ19A cleaved with PstI); 3) 0.1 fm HIV RNA; or 4) 0.1 fm β-globin DNA (Hβ19A cleaved with PstI in 100 µl containing 40 mM Tris-HCl, pH 8.1, 8 mM MgCl$_2$, 25 mM NaCl, 2 mM spermidine-(HCl)$_3$, 5 mM dithiothreitol, 100 µl each dATP, dTTP, dCTP and dGTP, 1 mM each rATP, rUTP, rCTP, and rGTP. 100 µg/ml BSA (nuclease-free), and 250 nM primer A

(SEQ ID No: 21) (5'-
AATTTAATACGACTCACTATAGGGACACCTAGGGCTAACTATGTGTCCTAATAAGG-3'),

250 nM primer B (SEQ ID No: 6) (5'-ACACCATATGTATGTTTCAGGGAAAGCTA-3'), 250 nM primer C

(SEQ ID No: 27)
(5'-TAATACGACTCACTATAGGGAACTAAAGGCACCGAGCACTTTCTTGCC-3'),

and 250 nM primer D ( SEQ ID No: 23) (5'-ACATTGCTTCTGACACAACTGTGTTCA-3'). A sample having 1/20th the starting nucleic acids was placed into denaturing solution 7.4% formaldehyde, 10x SSC (1.5 M NaCl, 0.15 M Na citrate, pH 7.4) for the zero time-point samples.

The samples were boiled for 1 minute and cooled at 42°C for 1 minute, followed by the addition of 10 units of AMV reverse transcriptase. The reactions are incubated at 42°C for 15 minutes, boiled for 1 minute, and cooled at 42°C for 1 minute. An additional 10 units of AMV reverse transcriptase are added, followed by incubation at 42°C for 15 minutes. One hundred units of T7 RNA polymerase are added, followed by incubation at 37°C for 30 minutes. This cycle is repeated a second time. (More cycles can be done, depending on the amplification needed.) Two samples containing 1/20th the starting target nucleic acids were placed into denaturing solution for the second transcription time-point samples.

All samples were heated to 55°C for 30 minutes prior to filtering onto two nitrocellulose membranes. The nucleic acids were fixed to the membrane by irradiation with 254 nm UV light for 4 minutes. As controls, 1, 0.1, and 0.01 fm of plasmid pARV7/2 [Luciw et al., Nature 312, 760 (1984)], which contains the entire HIV genome cDNA as well as plasmid Hβ19A [Wallace et al., Nucl. Acids Res. 9, 3647 (1981)], were denatured in 0.2 N NaOH, neutralized with an equal volume of 2 M ammonium acetate, and then filtered onto the nitrocellulose membrane. One membrane was hybridized with [32]p-labeled oligonucleotide 86-31[1], which is specific for the amplified product of HIV. The other mem-

brane was hybridized to [32]p-labeled oligonucleotide 87-459[2], which will hybridize to the amplified β-globin product, as well as to the target β-globin plasmid.

The hybridizations were in 1% SDS, 0.9 M NaCl, 50 nM $NaH_2PO_4$ (pH 7.4), 5 mM EDTA (pH 7.4), and $10^6$ cpm/m. [32]p-labeled oligonucleotide for 1 hour at 55°C. The membranes were washed 3 times in 1% SDS, 0.18 M NaCl, 10 mM $NaH_2PO_4$ (pH 7.4), and 1 mM EDTA at room temperature, followed by 1 wash in the same buffer at 55°C.

The membranes were then autoradiographed for 16 hours on Kodak XAR film at -70°C with one intensifying screen.

Notes

1. 86-31: (SEQ ID No: 28) 5'-GCACACAAGTAGACCCTGAACTAGCAGACCA-3'
2. 87-459: (SEQ ID No: 29) 5'-AGGTTTAAGGAGACCAATAGAAACT-3'

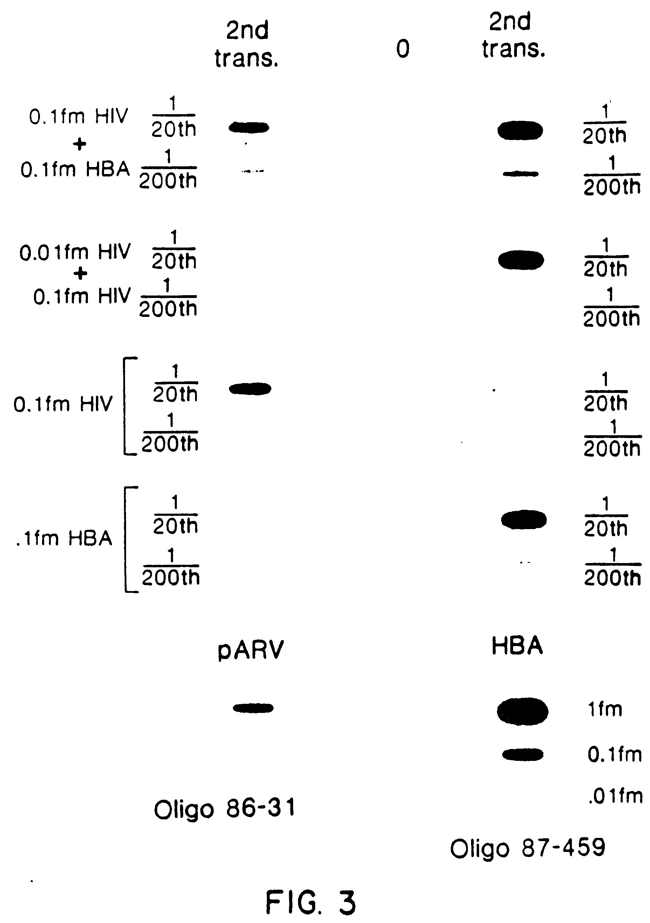

The autoradiograph in Figure 3 shows the amount of HIV and β-globin sequences detected after two cycles of TAS carried out simultaneously on HIV and β-globin nucleic acid. The starting amount of β-globin was kept constant while the amount of HIV target was varied.

**Claims**

1. A method for amplifying a target nucleic acid sequence in a sample containing nucleic acid comprising:

   (1) hybridizing to said target nucleic acid sequence a first single-stranded DNA primer, wherein said primer comprises a promoter sequence linked by a sequence of 0 to 100 nucleotides to a sequence of nucleotides which are complementary to the 3' end of said target sequence, said complementary sequence being at least 10 nucleotides in length,

   (2) extending said first primer hybridized in accordance with step (1) in a reaction catalysed by a DNA polymerase to form a first complementary DNA sequence,

   (3) rendering single-stranded the duplex formed in step (2),

   (4) hybridizing to said first complementary DNA sequence a second single-stranded DNA primer having a sequence of at least 10 nucleotides corresponding to the 5' end of said target sequence and optionally a further DNA sequence of up to 100 nucleotides,

   (5) extending said second primer hybridized in accordance with step (4) in a reaction catalysed by a DNA polymerase to form a second complementary DNA sequence and

   (6) employing the double-stranded DNA product of step (5) in the preparation of a plurality of RNA transcripts complementary to said target sequence in a reaction catalysed by a DNA-dependent RNA polymerase with specificity for the promoter comprised in said first primer.

2. A method for amplifying a target nucleic acid sequence as claimed in claim 1 wherein said target sequence is further amplified by

   (7) hybridizing to the RNA transcripts produced in step (6) a third single-stranded DNA primer wherein said primer comprises a promoter sequence linked by a sequence of 0 to 100 nucleotides to a sequence of nucleotides which are complementary to the 3' end of said target sequence, said complementary sequence being at least 10 nucleotides in length and optionally not, or not substantially, overlapping with the sequence of at least 10 nucleotides corresponding to the 5' end of said target sequence in said second primer, said promoter sequence being the same or different from the promoter sequence of said first DNA primer;

   (8) extending said third primer in a reaction catalysed by a DNA polymerase to form a third complementary DNA sequence;

   (9) rendering single - stranded the nucleic acid duplex formed in step (8);

(10) hybridizing to said third complementary DNA sequence a fourth single-stranded DNA primer having a sequence of at least 10 nucleotides corresponding to the 5' end of the RNA transcripts produced in step (6) and optionally a further DNA sequence of up to 100 nucleotides, said corresponding sequence optionally not, or not substantially, overlapping with the sequence of at least 10 nucleotides which are complementary to the 3' end of said target sequence in said first primer;

(11) extending said fourth primer in a reaction catalysed by a DNA polymerase to form a fourth complementary DNA and

(12) employing the double-stranded DNA product of step (5) in the preparation of a plurality of transcripts corresponding to said target sequence in a reaction catalysed by a DNA-dependent RNA polymerase with specificity for the promoter comprised in said third primer.

3. A method useful for detection of at least one specific nucleic acid target sequence in a sample containing nucleic acid comprising preparing, according to claim 1 or 2, RNA transcripts which contain a sequence complementary to or corresponding to said target sequence and detecting the presence of said RNA sequence.

4. A method according to Claim 3 wherein said transcripts contain a replicase recognition site for replication of said transcripts by replicase induction.

5. A method according to Claim 3 or Claim 4 wherein the detected RNA sequence of said RNA transcripts are measured in a standardized manner so as to measure the amount of target sequence contained in a sample of nucleic acid used in preparing the double-stranded nucleic acid template.

6. A method according to Claim 5 wherein the detected RNA sequence of said RNA transcripts is measured in a manner internally standardized with the presence of a known copy number of nucleic acid also contained in said sample.

7. A method according to Claim 3 wherein said nucleic acid sequence is a segment of a human immunodeficiency virus.

8. A method according to any of Claims 1 to 4 wherein the promoter corresponding to the promoter sequence of the first primer is a bacteriophage T7 promoter and the RNA transcripts are produced using T7 RNA polymerase.

9. A method according to any of Claims 1 to 4 wherein the promoter corresponding to the promoter sequence of the first primer is a bacteriophage SP6 promoter and the RNA transcripts are produced using SP6 RNA polymerase.

10. A method according to any of Claims 1 to 4 wherein the target sequence is a DNA sequence and the polymerase extension reaction is catalyzed by E. coli DNA polymerase 1.

11. A method according to any one of Claims 1 to 4 wherein the target sequence is a DNA sequence and the polymerase extension reaction is catalyzed by the Klenow Fragment of E. coli DNA polymerase I.

12. A method according to any one of Claims 1 to 4 wherein the target sequence is a DNA sequence and the polymerase extension reaction is catalyzed by T4 DNA polymerase.

13. A method according to any one of claims 1 to 4 wherein all polymerase extension reactions are catalyzed by a reverse transcriptase.

14. A method according to Claim 3 wherein said RNA transcripts are labelled prior to detection.

15. A method according to Claim 14 wherein said RNA transcripts are radio-labelled.

16. A method according to Claim 14 wherein said RNA transcripts are chromophore labelled.

17. A method according to Claim 13 wherein the reverse transcriptase is selected from the group consisting of AMV reverse transcriptase and MMLV reverse transcriptase.

**18.** A method according to any of Claims 1 to 17 wherein strands are separated by thermal denaturation.

**Patentansprüche**

**1.** Verfahren zur Vervielfachung einer Nukleinsäurezielsequenz in einer Nukleinsäure enthaltenden Probe, welches umfasst:

(1) Hybridisieren eines ersten Einzelstrang-Primers mit genannter Nukleinsäurezielsequenz, wobei der genannte Primer eine Promotersequenz umfasst, die durch eine Sequenz von 0 bis 100 Nukleotiden mit einer Sequenz verbunden ist, die zu dem 3ŒEnde der genannten Zielsequenz komplementär ist, wobei die genannte komplementäre Sequenz mindestens eine Länge von 10 Nukleotiden aufweist,

(2) Erstrecken des genannten ersten Primers in Uebereinstimung mit Schritt (1) in einer von einer DNA-Polymerase katalysierten Reaktion, um eine erste komplementäre DNA-Sequenz zu bilden,

(3) Herstellen von Einzelsträngen aus der in Schritt (2) gebildeten Duplex,

(4) Hybridisieren eines zweiten einzelsträngigen DNA-Primers, der eine Sequenz aus mindestens 10 des 5ŒEndes der Zielsequenz entsprechenden Nukleotiden umfasst, mit genannter ersten komplementären DNA-Sequenz, und gegebenenfalls einer weiteren Nukleotidsequenz mit bis zu 100 Nukleotiden,

(5) Erstrecken des genannten zweiten Primers in Uebereinstimmung mit Schritt (4) in einer von einer DNA-Polymerase katalysierten Reaktion, um eine erste komplementäre DNA-Sequenz zu bilden, und

(6) Verwenden des doppelsträngigen DNA-Produkts von Schritt (5) für die Herstellung einer Vielzahl von RNA-Transkripten, die zu der genannten Zielsequenz komplementär sind, in einer Reaktion, die von einer DNA-abhängigen RNA-Polymerase mit Spezifität für den in dem ersten Primer enthaltenden Promoter katalysiert wird.

**2.** Verfahren zur Vervielfachung einer Nukleinsäure-Zielsequenz nach Anspruch 1, worin die genannte Zielsequenz weiter vervielfacht wird durch

(7) Hybridisieren des in Schritt (6) hergestellten RNA-Transkripts mit einem dritten einzelsträngigen DNA-Primer, wobei der genannte Primer eine Promotersequenz umfasst, die durch eine Sequenz von 0 bis 100 Nukleotiden mit einer Sequenz von Nukleotiden verbunden ist, die zu dem 3ŒEnde der genannten Zielsequenz komplementär sind, wobei die genannte komplementäre Sequenz eine Länge von mindestens 10 Nukleotiden umfasst und gegebenenfalls nicht oder nicht wesentlich mit der Sequenz von mindestens 10 Nukleotiden überlappt, die dem 5ŒEnde der genannten Zielsequenz in dem genannten zweiten Primer entsprechen, wobei die Promotersequenz gleich oder verschieden von der Promotersequenz des genannten ersten Primers ist,

(8) Erstrecken des genannten dritten Primers in einer von einer DNA-Polymerase katalysierten Reaktion, um eine dritte komplentäre Sequenz zu bilden;

(9) Herstellen von Einzelsträngen aus der in Schritt (8) gebildeten Nukleinsäure-Duplex;

(10) Hybridisieren eines vierten Einzelstrang-DNA-Primers mit der genannten dritten komplementären DNA-Sequenz, wobei der genannte Primer eine Sequenz von mindestens 10 Nukleotiden umfasst, die dem 5ŒEnde des in Schritt (6) hergestellten RNA-Transkripts entspricht und gegebenenfalls mit einer weiteren DNA-Sequenz von bis zu 100 Nukleotiden, die gegebenenfalls nicht oder nicht wesentlich mit der Sequenz von mindestens 10 Nukleotiden überlappt, die zu dem 3ŒEnde der genannten Zielsequenz in dem genannten ersten Primer komplementär sind;

(11) Erstrecken des genannten vierten Primers in einer von einer DNA-Polymerase katalysierten Reaktion, um eine vierte komplementäre Sequenz zu bilden; und

(12) Verwenden des doppelsträngigen DNA-Produkts aus Schritt (5) zur Herstellung einer Vielzahl von Transkripten, die der genannten Zielsequenz entsprechen, in einer Reaktion, die von einer DNA-abhängigen RNA-Polymerase katalysiert wird, die eine Spezifität für den in genanntem dritten Primer enthaltenen Promoter aufweist.

3. Verfahren zum Nachweis von mindestens einer spezifischen Nukleinsäure-Zielsequenz in einer Nukleinsäure enthaltenden Probe, das Herstellen von RNA-Transkripten nach Anspruch 1 oder 2 umfasst, welche eine Sequenz enthalten, die zu der genannten Zielsequenz komplementär ist oder dieser entspricht und Nachweisen der Anwesenheit der genannten RNA-Sequenz.

4. Verfahren gemäss Anspruch 3, dadurch gekennzeichnet, dass die genannten Transkripte eine Replikase-Erkennungsstelle für die Replikation der genannten Transkripte durch Replikase-Induktion enthalten.

5. Verfahren gemäss Anspruch 3 oder 4, dadurch gekennzeichnet, dass die nachgewiesene RNA-Sequenz der genannten RNA-Transkripte auf standardisierte Weise gemessen wird, um die Menge der in einer Nukleinsäureprobe enthaltenen Zielsequenz zu messen, welche zum Herstellen der doppelsträngigen Nukleinsäure-Matrizen verwendet wird.

6. Verfahren gemäss Anspruch 5, dadurch gekennzeichnet, dass die nachgewiesene RNA-Sequenz der genannten RNA-Transkripte auf eine Weise gemessen wird, bei der durch die Anwesenheit einer bekannten Kopienzahl von Nukleinsäuren, die ebenfalls in der genannten Probe enthalten sind, intern standardisiert wird.

7. Verfahren gemäss Anspruch 3, dadurch gekennzeichnet, dass die genannte Nukleinsäure ein Segment des menschlichen Immunschwächevirus ist.

8. Verfahren gemäss einem der Ansprüche 1 bis 4, dadurch gekennzeichnet, dass der Promoter, der der Promotersequenz des ersten Primers entspricht, ein Bacteriophagen-T7-Promoter ist und die RNA-Transkripte unter Verwendung von T7-RNA-Polymerase hergestellt werden.

9. Verfahren gemäss einem der Ansprüche 1 bis 4, dadurch gekennzeichnet, dass der Promoter, der der Promotersequenz des ersten Primers entspricht, ein Bacteriophagen-SP6-Promoter ist und die RNA-Transkripte unter Verwendung von SP6-RNA-Polymerase hergestellt werden.

10. Verfahren gemäss einem der Ansprüche 1 bis 4, dadurch gekennzeichnet, dass die Zielsequenz eine DNA-Sequenz ist und die Polymeraseextensionsreaktion durch E.coli-Polymerase I katalysiert wird.

11. Verfahren gemäss einem der Ansprüche 1 bis 4, dadurch gekennzeichnet, dass die Zielsequenz eine DNA-Sequenz ist und die Polymeraseextensionsreaktion durch das Klenow-Fragment der E.coli-Polymerase I katalysiert wird.

12. Verfahren gemäss einem der Ansprüche 1 bis 4, dadurch gekennzeichnet, dass die Zielsequenz eine DNA-Sequenz ist und die Polymeraseextensionsreaktion durch T4-DNA-Polymerase katalysiert wird.

13. Verfahren gemäss einem der Ansprüche 1 bis 4, dadurch gekennzeichnet, dass alle Polymeraseextensionsreaktionen durch Reverse Transkriptase katalysiert werden.

14. Verfahren gemäss Anspruch 3, dadurch gekennzeichnet, dass die genannten RNA-Transkripte vor dem Nachweis markiert werden.

15. Verfahren gemäss Anspruch 14, dadurch gekennzeichnet, dass die genannten RNA-Transkripte radio-markiert werden.

16. Verfahren gemäss Anspruch 14, dadurch gekennzeichnet, dass die genannten RNA-Transkripte chromophor-markiert sind.

17. Verfahren gemäss Anspruch 13, worin die Reverse Transkriptase aus der Gruppe ausgewählt wird, bestehend aus AMV-Reverser Transkriptase und MMLV-Reverser Transkriptase.

18. Verfahren gemäss einem der Ansprüche 1 bis 17, worin die Stränge durch thermische Denaturierung getrennt werden.

**Revendications**

1. Procédé d'amplification d'une séquence d'acide nucléique cible dans un échantillon contenant un acide nucléique comprenant :

   (1) l'hybridation à ladite séquence d'acide nucléique cible d'une première amorce d'ADN simple brin, ladite amorce comprenant une séquence promoteur liée par une séquence de 0 à 100 nucléotides à une séquence de nucléotides qui est complémentaire de l'extrémité 3' de ladite séquence cible, ladite séquence complémentaire ayant une longueur d'au moins 10 nucléotides.
   (2) la prolongation de ladite première amorce hybridée conformément à l'étape (1) dans une réaction catalysée par une ADN polymérase pour former une première séquence d'ADN complémentaire.
   (3) la formation de molécules simple brin à partir du duplex d'acide nucléique formé à l'étape (2).
   (4) l'hybridation à ladite première séquence d'ADN complémentaire d'une seconde amorce d'ADN simple brin possédant une séquence d'au moins 10 nucléotides correspondant à l'extrémité 5' de ladite séquence cible, et éventuellement une autre séquence d'ADN allant jusqu'à 100 nucléotides.
   (5) la prolongation de ladite seconde amorce hybridée conformément à l'étape (4) dans une réaction catalysée par une ADN polymérase pour former une seconde séquence d'ADN complémentaire, et
   (6) l'utilisation de l'ADN double brin produit à l'étape (5) dans la préparation d'une pluralité de transcrits d'ARN complémentaires de ladite séquence cible dans une réaction catalysée par une ARN polymérase ADN-dépendante possédant une spécificité pour le promoteur compris dans ladite première amorce.

2. Un procédé d'amplification d'une séquence d'acide nucléique cible conformément à la revendication 1, dans lequel ladite séquence cible est de surcroît amplifiée en procédant aux étapes suivantes :

   (7) une hybridation aux transcrits d'ARN produits à l'étape (6) d'une troisième amorce d'ADN simple-brin, ladite amorce comprenant une séquence promoteur liée par une séquence de 0 à 100 nucléotides à une séquence de nueléotides qui est complémentaire de l'extrémité 3' de ladite séquence cible, ladite séquence complémentaire ayant une longueur d'au moins 10 nucléotides, et présentant éventuellement un chevauchement pratiquement inexistant, voire nul, avec la séquence d'au moins 10 nucléotides correspondant à l'extrémité 5' de ladite séquence cible dans ladite seconde amorce, ladite séquence promoteur étant identique à ou différente de la séquence promoteur de ladite première amorce d'ADN ;
   (8) la prolongation de ladité troisième amorce dans une réaction catalysée par une ADN polymérase pour former une troisième séquence d'ADN complémentaire ;
   (9) la transformation du duplex d'acide nucléique formé à l'étape (8), en simple brin :
   (10) l'hybridation à ladite troisième séquence d'ADN complémentaire d'une quatrième amorce d'ADN simple brin possédant une séquence d'au moins 10 nucléotides correspondant à l'extrémité 5' des transcrits d'ARN produits à l'étape (4) et éventuellement une autre séquence d'ADN allant jusqu'à 100 nucléotides, ladite séquence correspondante présentant éventuellement un chevauchement pratiquement inexistant, voire nul, avec la séquence d'au moins 10 nucléotides qui est complémentaire à l'extrémité 3' de ladite séquence cible dans ladite première amorce ;
   (11) la prolongation de ladite quatrième amorce dans une réaction catalysée par une ADN polymérase pour former une quatrième séquence d'ADN complémentaire, et
   (12) l'utilisation de l'ADN double brin produit à l'étape (5) dans la préparation d'une pluralité de transcrits d'ADN correspondant à ladite séquence cible dans une réaction catalysée par une ARN polymérase ADN dépendante possédant une spécificité pour le promoteur compris dans ladite troisième amorce.

3. Un procédé utile pour la détection d'au moins une séquence cible d'acide nucléique spécifique dans un échantillon contenant un acide nucléique comprenant la préparation, conformément à la revendication 1 ou 2, de transcrits d'ARN qui contiennent une séquence complémentaire de ou correspondant à ladite séquence cible et la détection de la présence de ladite séquence d'ARN.

4. Un procédé selon la revendication 3, dans lequel lesdits transcrits contiennent un site de reconnaissance de la réplicase pour la réplication desdits transcrits par induction de la réplicase.

5. Un procédé selon la revendication 3 ou la revendication 4 dans lequel la séquence d'ARN détectée desdits transcrits d'ARN est dosée d'une manière normalisée de manière à évaluer la quantité de séquence cible présente dans un échantillon d'acide nucléique utilisé dans la préparation de la matrice d'acide nucléique double brin.

6. Un procédé selon la revendication 5 dans lequel la séquence d'ARN détectée desdits transcrits d'ARN est dosée d'une manière avec référence interne constituée par la présence d'un nombre connu d'exemplaires d'un acide nucléique également présent dans ledit échantillon ;

7. Un procédé selon la revendication 3 dans lequel ladite séquence d'acide nucléique est un segment d'un virus de l'immunodéficience humaine.

8. Un procédé selon l'une quelconque des revendications 1 à 4 dans lequel le promoteur correspondant à la séquence promoteur de la première amorce est un promoteur du bactériophage T7 et les transcrits d'ARN sont produits en utilisant l'ARN polymérase T7.

9. Un procédé selon l'une quelconque des revendications 1 à 4 dans lequel le promoteur correspondant à la séquence promoteur de la première amorce est un promoteur du bactériophage SP6 et les transcrits d'ARN sont produits en utilisant l'ARN polymérase SP6.

10. Un procédé selon l'une quelconque des revendications 1 à 4 dans lequel la séquence cible est une séquence d'ADN et la réaction d'extension par polymérase est catalysée par l'ADN polymérase I d'E. coli.

11. Un procédé selon l'une quelconque des revendications 1 à 4 dans lequel la séquence cible est une séquence d'ADN et la réaction d'extension par polymérase est catalysée par le fragment de Klenow de l'ADN polymérase I d'E. coli.

12. Un procédé selon l'une quelconque des revendications 1 à 4 dans lequel la séquence cible est une séquence d'ADN et la réaction d'extension par polymérase est catalysée par l'ADN polymérase de T4.

13. Un procédé selon l'une quelconque des revendications 1 à 4 dans lequel toutes les réactions d'extension par une polymérase sont catalysées par une transcriptase inverse.

14. Un procédé selon la revendication 3 dans lequel lesdits transcrits d'ARN sont marqués avant la détection.

15. Un procédé selon la revendication 14 dans lequel lesdits transcrits d'ARN sont marqués avec un isotope radioactif.

16. Un procédé selon la revendication 14 dans lequel lesdits transcrits d'ARN sont marqués avec un chromophore.

17. Un procédé selon la revendication 13 dans lequel la transcriptase inverse est sélectionnée parmi le groupe constitué par la transcriptase inverse de l'AMV et la transcriptase inverse du MMLV.

18. Un procédé selon l'une quelconque des revendications 1 à 17 dans lequel les brins sont séparés par dénaturation thermique.

FIG. IA

FIG. IB

2ND COMPLEMENTARY DNA
(VARIABLE SUBSEGMENT) 1c
(PROMOTER) 1c

(PROMOTER) 1
(VARIABLE SUBSEGMENT) 1
1ST COMPLENETARY DNA

3'
5'

(VARIABLE SUBSEGMENT) 2
2ND COMPLEMENTARY DNA

1ST COMPLEMENTARY DNA

5'
3'

((1ST SUBSEGMENT)$\textit{t}_c$
(2ND SUBSEGMENT)$\textit{t}_c$
(3RD SUBSEGMENT)$\textit{t}_c$

TRANSCRIBE WITH BACTERIOPHAGE DNA-DEPENDENT
RNA POLYMERASE WHICH RECOGNIZES (PROMOTER) 1

(1ST SUBSEGMENT)$\textit{t}_{cr}$
(1ST SUBSEGMENT)$\textit{t}_{2cr}$
(VARIABLE SUBSEGMENT)$_{1r}$

(1ST SUBSEGMENT)$\textit{t}_{cr}$
(2ND SUBSEGMENT)$\textit{t}_{cr}$

(3RD SUBSEGMENT)

(3RD SUBSEGMENT)$\textit{t}_{cr}$
(3RD SUBSEGMENT)$\textit{t}_{1cr}$
(3RD SUBSEGMENT)$\textit{t}_{2cr}$
(VARIABLE SUBSEGMENT)$_{2cr}$

5'
3' ~ RNA 1

FIG. 1C

30

FIG. 2A

FIG. 2B

FIG. 2C

EP 0 623 683 B1

FIG. 3

# FIG. 4

RNA Polymerase (30'-45')

$(10^2 - 10^3)$

3' Qβ

5' Qβ or BMV Sequence

RNA Replicase (10'-30')

$(10^2 - 10^6)$

EP 0 623 683 B1